# EUROPÄISCHE PATENTSCHRIFT

(11) **EP 1 516 059 B1**
(45) Veröffentlichungstag und Bekanntmachung des Hinweises auf die Patenterteilung: **28.03.2012**
(21) Anmeldenummer: 03732449.8
(22) Anmeldetag: 23.05.2003
(51) Int. Cl.: C12P 13/12, C12N 9/10

(54) **VERFAHREN ZUR FERMENTATIVEN HERSTELLUNG SCHWEFELHALTIGER FEINCHEMIKALIEN**
METHOD FOR THE PRODUCTION OF SULPHUR-CONTAINING FINE CHEMICALS BY FERMENTATION
PROCEDE POUR PRODUIRE PAR FERMENTATION DES PRODUITS DE CHIMIE FINE CONTENANT DU SOUFRE

(30) Priorität: 23.05.2002 DE 10222858
(43) Veröffentlichungstag der Anmeldung: 23.03.2005
(73) Patentinhaber: Evonik Degussa GmbH, 45128 Essen (DE)
(72) Erfinder: SCHRÖDER, Hartwig, 69226 Nussloch (DE); KRÖGER, Burkhard, 67117 Limburgerhof (DE); ZELDER, Oskar, 67346 Speyer (DE); KLOPPROGGE, Corinna, 67065 Ludwigshafen (DE); HÄFNER, Stefan, 67063 Ludwigshafen (DE)
(86) Internationale Anmeldenummer: PCT/EP2003/005423
(87) Internationale Veröffentlichungsnummer: WO 2003/100072

(56) Entgegenhaltungen:
- WO-A-01/00843
- WO-A-02/10209
- WO-A-03/023044
- RECZKOWSKI R S ET AL: "Structural and functional roles of cysteine 90 and cysteine 240 in S-adenosylmethionine synthetase." THE JOURNAL OF BIOLOGICAL CHEMISTRY, vol. 270, no. 31, 4 August 1995 (1995-08-04), pages 18484-18490, XP002260183 ISSN: 0021-9258
- DATABASE WPI Section Ch, Week 200103 Derwent Publications Ltd., London, GB; Class B05, AN 2001-018703 XP002260186 -& JP 2000 139471 A (AJINOMOTO KK) 23 May 2000 (2000-05-23)
- NAKAMORI S ET AL: "Mechanism of L-methionine overproduction by Escherichia coli: The replacement of Ser-54 by Asn in the MetJ protein causes the derepression of L-methionine biosynthetic enzymes" APPLIED MICROBIOLOGY AND BIOTECHNOLOGY, vol. 52, no. 2, August 1999 (1999-08), pages 179-185, XP002242975 ISSN: 0175-7598
- SHEN BO ET AL: "High free-methionine and decreased lignin content result from a mutation in the Arabidopsis S-adenosyl-L-methionine synthetase 3 gene." THE PLANT JOURNAL: FOR CELL AND MOLECULAR BIOLOGY, vol. 29, no. 3, February 2002 (2002-02), pages 371-380, XP002260184 ISSN: 0960-7412
- GOTO D B ET AL: "A single-nucleotide mutation in a gene encoding S-adenosylmethionine synthetase is associated with methionine over-accumulation phenotype in Arabidopsis thaliana" GENES AND GENETIC SYSTEMS, vol. 77, no. 2, April 2002 (2002-04), pages 89-95, XP002242977 ISSN: 1341-7568
- GROSSMANN K ET AL: "Rapid cloning of metK encoding methionine adenosyltransferase from Corynebacterium glutamicum by screening a genomic library on a high density colony-array" FEMS MICROBIOLOGY LETTERS, vol. 193, no. 1, 1 December 2000 (2000-12-01), pages 99-103, XP000984551 ISSN: 0378-1097
- PATENT ABSTRACTS OF JAPAN vol. 1995, no. 11, 26 December 1995 (1995-12-26) -& JP 07 227287 A (MITSUBISHI CHEM CORP), 29 August 1995 (1995-08-29) cited in the application
- SEKOWSKA A ET AL: "Sulfur metabolism in Escherichia coli and related bacteria: Facts and fiction" JOURNAL OF MOLECULAR MICROBIOLOGY AND BIOTECHNOLOGY, vol. 2, no. 2, April 2000 (2000-04), pages 145-177, XP002242976 ISSN: 1464-1801
- WEI YUHONG ET AL: "Studies on the role of the metK gene product of Escherichia coli K-12." MOLECULAR MICROBIOLOGY, vol. 43, no. 6, March 2002 (2002-03), pages 1651-1656, XP002260185 ISSN: 0950-382X cited in the application
- KASE H ET AL: "Isolation and characterization of S-adenosylmethionine-requiring mutants and role of S-adenosylmethionine in the regulation of methionine biosynthesis in Corynebacterium glutamicum", AGR. BIOL. CHEM., vol. 39, no. 1, 1975, pages 161-168,
- DATABASE UNIPROT 01 October 1996 'METK_BACSU'

## Beschreibung

Gegenstand der Erfindung ist neues Verfahren zur fermentativen Herstellung von L-Methionin bei dem Bakterien genutzt werden, in denen Nukleotidsequenzen exprimiert werden, die für Mutanten der S-Adenosylmethionin-Synthase (metK) (E.C.2.5.1.6) kodieren; Nukeotidsequenzen, welche für diese Mutanten kodieren, sowie die damit transformierten rekombinanten Mikroorganismen, sowie neuartige metK-Mutanten mit veränderter Enzymaktivität.

### Stand der Technik

Schwefelhaltige Feinchemikalien, wie zum Beispiel Methionin, Homocystein, S-Adenosyl-Methionin, Glutathion, Cystein, Biotin, Thiamin, Liponsäure werden über natürliche Stoffwechselprozesse in Zellen hergestellt und werden in vielen Industriezweigen verwendet, einschließlich der Nahrungsmittel-, Futtermittel-, Kosmetik- und pharmazeutischen Industrie. Diese Substanzen, die zusammen als "schwefelhaltige Feinchemikalien" bezeichnet werden, umfassen organische Säuren, sowohl proteinogene als auch nicht-proteinogene Aminosäuren, Vitamine und Cofaktoren. Ihre Produktion erfolgt am zweckmäßigsten im Großmaßstab mittels Anzucht von Bakterien, die entwickelt wurden, um große Mengen der jeweils gewünschten Substanz zu produzieren und sezemieren. Für diesen Zweck besonders geeignete Organismen sind die gram-positiven, nicht-pathogenen coryneformen Bakterien.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen, wie zum Beispiel Rührung und Versorgung mit Sauerstoff, oder die Zusammensetzung der Nährmedien, wie zum Beispiel die Zuckerkonzentration während der Fermentation, oder die Aufarbeitung zum Produkt, beispielsweise durch lonenaustauschchromatographie, oder die intrinsischen Leistungseigenschaften des Mikroorganismus selbst betreffen.

Über Stammselektion sind eine Reihe von Mutantenstämmen entwickelt worden, die ein Sortiment wünschenswerter Verbindungen aus der Reihe der schwefelhaltigen Feinchemikalien produzieren. Zur Verbesserung der Leistungseigenschaften dieser Mikroorganismen hinsichtlich der Produktion eines bestimmten Moleküls werden Methoden der Mutagenese, Selektion und Mutantenauswahl angewendet. Dies ist jedoch ein zeitaufwendiges und schwieriges Verfahren. Auf diese Weise erhält man z.B. Stämme, die resistent gegen Antimetabolite, wie z. B. die Methionin-Analoga α-Methyl-Methionin, Ethionin, Norleucin, N-Acetylnorleucin, S-Triftuoromethylhomocystein, 2-amin-5-heptencarbonsäure, Seleno-Methionin, Methioninsulfoximin, Methoxin; 1-Aminocydopentan-Carbonsäure oder auxotroph für regulatorisch bedeutsame Metabolite sind und schwefelhaltige Feinchemikalien, wie z. B. L-Methionin, produzieren.

Seit einiges Jahren werden ebenfalls Methoden der rekombinanten DNA-Techhik zur Stammverbesserung von L-Aminosäure produzierender Stämme von Corynebacterium eingesetzt, indem man einzelne Aminosäure-Biosynthesegene amplifiziert und die Auswirkung auf die Aminosäure-Produktion untersucht.

Aus oder JP-A-06-020809 ist eine Nukleotidsequenz für ein S-Adenosylmethionin kodierendes Gen aus Brevibacterium flavum MJ-233, einem coryneformen Bakterium, bekannt. Die korrespondierende Aminosäuresequenz umfässt 412 Aminosäuren. Das Protein weist unter anderem in den Positionen 24 und 94 jeweils einen Cysteinrest auf, welche in den entsprechenden Enzymen zahlreicher anderer coryneförmer Bakterien konserviert sind. Die offenbarte Aminosäuresequenz besitzt einen charakteristischen Sequenzäbschhitt zwischen den Resten 137 und 154. Die Herstellung von Mutanten und deren Verwendung bei der fermentativen Herstellung schwefelhaltiger Feinthemikalien ist dann nicht beschrieben

Aus der WO-A-01/00843 ist ein metK-Gen aus C. glutamicum bekannt, welches für ein Protein mit 407 Aminosäuren kodiert und eine Sequenz gemäß SEQ ID NO:16 aufweist.

Verbesserungen der fermentativen Herstellung von Feinchemikalien korrelieren in der Regel mit Verbesserungen von Stoffflüssen und Ausbeuten. Wichtig dabei ist es, Zwischen- oder Endprodukthemmungen Wichtiger 'Syntheseenzyme zu verhindern oder zu verringern. Ebenso ist es von Vorteil. Abflüsse des Kohlenstoffflüsses in ungewünschte Produkte oder Seitenprodukte zu verhindern oder zu verringem.

Der Einfluss von Stoffwechselmetaboliten auf die enzymatischen Aktivitäten von Stoffwechsetenzymen kann untersucht werden. Beispiele für solche Enzyme können metA, metB, metC; MetY, metH, metE, metF und weitere Enzyme im Stoffwechsel von Mikroorgamismen sein. Ein wichtige Stoffwechselprodukt des Methionins und damit ein wesentlicher Abfluß ist S-Adenosylmethionin.

Gleichzeitig ist S-Adenosylmethionin aber auch ein entscheidender Regulator der Methioninbiosynthese. Es ist zum Beispiel bekannt, dass die Biosynthese von L-Methionin in E. coli durch S-Adenosylmethionin inhibiert wird. S-Adenosylmethionin wirkt dort als ein Co-Repressor des Repressors metJ (Weissbach, H. Brot, N. (1991) Mol Microbiol. 5 (7), 1593-1597).

Die Synthese des S-Adenosylmethionins ist gleichzeitig ein wesentlicher Abfluß des gewünschten Wertproduktes L-Methionin. Deshalb ist es aus mehreren Gründen wünschenswert, die Menge des gebildeten S-Adenosylmethionins zu verringem:
a) die Menge des gebildeten L-Methionins würde erhöht,
b) die Repression von Genen der Methionin-Biosynthese verringert und
c) die Feedback-Inhibition von Enzymen der Methionin-Biosynthese würde verringert.

Die Deletion des metK Gens wäre der einfachste Weg, die Bildung des S-Adenosylmethionin zu verhindem. In Wei, Y. und Newman, E.B. (2002) Mol. Microbiol. 43 (6), 1651-1656 wird metK aber als ein essentielles Gen beschrieben und scheint somit für den Fachmann als Ansatzpunkt für ein verbesserte fermentativer Herstellung von schwefelhaltigen Feinchemikalien, insbesondere von L-Methionin auszuscheiden. GROSSMANN K ET AL: "Rapid cloning of metK encoding methionine adenosyltransferase from Corynebacterium glutamicum by screening a genomic library on a high density colonyarray", FEMS MICROBIOLOGY LETTERS, Bd. 193, Nr. 1, 1. Dezember 2000, Seiten 99-103 offenbaren sowohl die für metK kodierende Nukleotidsequenz aus Corynebacterium glutamicum, als auch deren Exprimierung in C. glutamicum. Die Nukleotidsequenz kodiert aber nicht für ein Polypeptid mit verringerter metK-Aktivität.
RECZKOWSKI R S ET AL: "Structural and functional roles of cysteine 90 and cysteine 240 in S-adenosylmethionine synthetase", THE JOURNAL OF BIOLOGICAL CHEMISTRY, Bd. 270, Nr. 31, 4. August 1995, Seiten 18484-18490 beschreiben metK-Mutanten mit verringerter metK-Aktivität, in der Cys90, gehörend zur Aminosäureteilsequenz GFDANSCA von metK aus Escherichia coli, durch Ala oder Ser ersetzt worden ist, ebenso wie für C90A/S-MetK kodierende Polynukleotide und Expressionskonstrukte.
KASE H ET AL: "Isolation and characterization of S-adenosylmethionine-requiring mutants and role of S-adenosylmethionine in the regulation of methionine biosynthesis in Corynebacterium glutamicum", AGR. BIOL. CHEM., Bd. 39, Nr. 1, 1975, Seiten 161-168 beschreiben ein Verfahren zur erhöhten fermentativen Herstellung von L-Methionin offenbart, das gekennzeichnet wird durch Fermentation einer C. glutamicum Mutante mit verringertem S-Adenosylmethionin Synthase-Aktivität.

### Kurze Beschreibung der Erfindung

Der Erfindung liegt daher die Aufgabe zugrunde, ein neues Verfahren zur verbesserten fermentativen Herstellung von L-Methionin und die dafür erforderlichen Mittel bereitzustellen.

Gelöst wird obige Aufgabe überraschenderweise durch Bereitstellung eines Verfahrens zur fermentativen Herstellung von L-Methionin, umfassend die Expression einer metK Nukleotidsequenz in einem coryneformen Bakterium, wobei die Nukleotidsequenz für eine S-Adenosylmethionin Synthase Mutante kodiert, deren Aktivität gegenüber dem Wildtyp Enzym verringert, ist. Beispielsweise ist die Mutante von der S-Adenosylmethionin Synthase aus Corynebacterium glutamicum abgeleitet und zeigt, gemessen in Corynebacterium glutamicum, eine geringere Aktivität als das Wildtypenzym.

Ein erster Gegenstand der Erfindung betrifft ein Verfahren zur fermentativen Herstellung von L-Methionin, welches folgende Schritte umfasst:
a) Fermentation einer L-Methionin produzierenden coryneformen Bakterienkultur, wobei in den coryneformen Bakterien zumindest eine Nukleotidsequenz exprimiert wird, welche für ein Protein mit verringerter Adenosylmethionin Synthese (metK)-Aktivität kodiert;
b) Anreicherung der schwefelhaltigen Feinchemikalie im Medium und/oder in den Zellen der Bakterien, und
c) Isolieren der schwefelhaltigen Feinchemikalie, welche vorzugsweise L-Methionin umfasst.

Gemäß einer bevorzugte Ausführungsform besitzt das mutierte coryneforme Bakterium außerdem eine, im Vergleich zum nichtmutierten Wildtyp, verbesserte metY Aktivität und/oder eine gesteigerte L-Methionin Menge (z. B. in g/l Fermentationsbrühe).

Im erfindungsgemäßen Verfahren wird als metK-kodierende Sequenz, insbesondere eine kodierte Nukleotidsequenz verwendet, welche für ein Protein mit verringerter metK-Aktivität kodiert, in welcher wenigstens ein Cysteinrest des Wildtypproteins substituiert ist.

Die metK-kodierende Sequenz kodiert für ein Protein mit metK-Aktivität, welches folgende Aminosäureteilsequenz gemäß SEQ ID NO:23 aufweist:

G(F/Y)(D/S) X¹X²(S/T)X³(G/_{A})_{V}

worin
X¹ und X² unabhängig voneinander für eine beliebige Aminosäure stehen; und
x³ für eine von Cys verschiedene Aminosäure steht. Besonders bevorzugt ist ein Verfahren gemäß obiger Definition, bei welchem die metK-kodierende Sequenz für ein Protein mit metK-Aktivität kodiert, wobei das Protein eine Aminosäuresequenz von Met1 bis Ala407 gemäß SEQ ID NO: 22 oder eine dazu homologe Aminosäuresequenz, welche für ein Protein mit funktionaler Äquivalenz steht, umfasst.

Die erfindungsgemäß eingesetzte metK-kodierende Sequenz umfasst vorzugsweise eine
kodierende Sequenz gemäß SEQ ID NO: 21 oder eine dazu homologe Nukleotidsequenz, welche für ein Protein mit metK-Aktivität kodiert.

Die kodierende metK-Sequenz ist eine in coryneformen Bakterien replizierbare oder eine stabil in das Chromosom integrierte DNA oder eine RNA.

Gemäß einer bevorzugten Ausführungsform wird das erfindungsgemäße Verfahren durchgeführt, indem man
a) einen mit einem Plasmidvektor transformierten Bakterienstamm einsetzt, der wenigstens eine Kopie der kodierenden metK-Sequenz unter der Kontrolle regulativer Sequenzen trägt, oder
b) einen Stamm einsetzt, in dem die kodierende metK-Sequenz in das Chromosom des Bakteriums integriert wurde.

Besonders bevorzugt sind Stämme obiger Definition in denen zusätzlich die Aktivität des metK-Wildtyp-Enzyms vollständig oder teilweise entfernt wurde, wie z.B. durch Deletion der kodierenden Sequenz des Wildtypenzyms.

Außerdem kann es wünschenswert sein, Bakterien zu fermentieren, in denen zusätzlich wenigstens ein weiteres Gen des Biosyntheseweges der gewünschten schwefelhaltigen Feinchemikalie verstärkt ist; und **/** oder in denen wenigstens ein Stoffwechselweg zumindest teilweise ausgeschaltet sind, der die Bildung von L-Methionin verringert.

Gemäß einer weiteren Ausführungsform des erfindungsgemäßen Verfahrens werden deshalb coryneforme Bakterien fermentiert, in denen gleichzeitig wenigstens eines der Gene, ausgewählt unter
1) dem für eine Aspartat Kinase kodierenden Gen lysc,
2) dem für eine Aspartat-Semialdehyd-Dehydrogenase-kodierenden Gen asd
3) dem für die Glycerinaldehyd-3-Phosphat Dehydrogenase kodierenden Gen gap,
4) dem für die 3-Phosphoglycerat Kinase kodierenden Gen pgk,
5) dem für die Pyruvat Carboxylase kodierenden Gen pyc,
6) dem für die Triosephosphat Isomerase kodierenden Gen tpi,
7) dem für die Homoserin O-Acetyltransferase kodierenden Gen metA,
8) dem für die Cystathionin-gamma Synthase kodierenden Gen metB,
9) dem für die Cystathionin-gamma Lyase kodierenden Gen metC,
10) dem für die Methionin Synthase kodierenden Gen metH,
11) dem für die Serin Hydroxymethyltransferase kodierenden Gen glyA,
12) dem für die O-Acetylhomoserin Sulfhydrylase kodierenden Gen metY,
13) dem für die Methylen-Tetrahydrofolat Reduktase kodieren Gen, metF
14) dem für die Phosphoserin Aminotransferase kodieren Gen serC
15) dem für die Phosphoserin Phosphatase kodieren Gen serB,
16) dem für die Serine Acetyl Transferase kodieren Gen cysE,
17) dem für die Cystein Synthase kodierenden Gen cysK,
18) dem für die Homoserin Dehydrogenase kodierenden Gen hom, überexprimiert ist.

Gemäß einer anderen Ausführungsform des erfindungsgemäßen Verfahrens werden coryneforme Bakterien fermentiert, in denen gleichzeitig wenigstens eines der Gene ausgewählt unter Genen der oben genannten Gruppe 1) bis 18) so mutiert ist, dass die korrespondierenden Proteine, verglichen mit nicht mutierten Proteinen, in geringerem Maße oder nicht durch Stoffwechselmetabolite in ihrer Aktivität beeinflusst werden und dass insbesondere die erfindungsgemäße Produktion von L-Methionin nicht beeinträchtigt wird, oder so dass ihre spezifische enzymatische Aktivität gesteigert wird:

Gemäß einer anderen Ausführungsform des erfindungsgemäßen Verfahrens werden coryneforme Bakterien fermentiert, in denen gleichzeitig wenigstens eines der Gene, ausgewählt unter
19) dem für die HomoserineKinase kodierenden Gen thrB,
20) dem für die Threonin Dehydratase kodierenden Gen ilvA,
21) dem für die Threonin Synthase kodierenden Gen thrC
22) dem für die Meso-Diaminopimelat D Dehydrogenase kodierenden Gen ddh
23) dem für die Phosphoenolpyruvat Carboxykinase kodierenden Gen pck,
24) dem für die Glucose-6-Phosphat-lsomerase kodierenden Gen pgi,
25) dem für die Pyruvat Oxidase kodierenden Gen poxB,
26) dem für die Dihydrodipicolinat Synthase kodiemden Gen dapA,
27) dem für die Dihydrodipicolinat Reduktase kodiemden Gen dapB; oder
28) dem für die Diaminopicolinat Decarboxylase kodiernden Gen lysA abschwächt ist, insbesondere durch Verringerung der Expressionsrate des korrespondierenden Gens.

Gemäß einer anderen Ausführungsform des erfindungsgemäßen Verfahrens werden coryneforme Bakterien fermentiert, in denen gleichzeitig wenigstens eines der Gene der obigen Gruppen 19) bis 28) mutiert ist, so dass die enzymatische Aktivität des korrespondierenden Proteins teilweise oder vollständig verringert wird.

Vorzugsweise werden in dem erfindungsgemäßen Verfahren Mikroorganismen der Art Corynebacterium glutamicum eingesetzt

Ein weiterer Gegenstand der Erfindung betrifft ein Verfahren zur Herstellung eines L-Methionin-haltigen Terfuttermittel-Additivs aus Fermentationsbrühen, welches folgende Schritte umfasst
a) Kultivierung und Fermentation eines L-Methionin produzierenden Mikroorganismus mit verringerter metK-Aktivität gemäß obiger Definition, in einem Fermentationsmedium:
b) Entfernung von Wasser aus der L-Methionin haltigen Fermentationsbrühe;
c) Entfernung der während der Fermentation gebildeten Biomasse in einer Menge von 0 bis 100 Gew.%; und
d) Trocknung-der gemäß b) und/oder c) erhaltenen Fermentationsbrühe, um das Tierfuttermittet-Additiv in der gewünschten Pulver- oder Granulatform zu erhalten.

Die Beichreibung betrifft außerdem isolierte Polynukleotide, die für ein Polypeptid mit verringerter metK-Aktivität gemäß obiger Definition kodieren; sowie metK-Mutanten mit verringerter Aktivität, welche von diesen Polynukleotiden kodiert werden.

Gegenstand der Beichreibung sind außerdem rekombinante coryneforme Bakterien, die ein mutiertes metK Gen gemäß obige Definition exprimieren und insbesondere solche rekombinante coryneforme Bakterien welche das metK-Wildtypenzym nicht mehr exprimieren.

Rekombihante coryneforme Bakterien zeigen im Vergleich zum korrespondierenden Wildtypstamm wenigstens eines der folgenden Merkmale:
a) geringeren intrazellulären S-Adenosylmethionin Titer
b) geringere intrazelluläre S-Adenosylmethionin Synthase Konzentration, oder
c) geringere Aktivität der S-Adenosylmethionin Synthase, bestimmt anhand der S-Adenosylmethionin Bildungsrate;
und zusätzlich gegebenenfalls wenigstens eines der folgenden Merkmale:
d) verbesserte metY Aktivität, oder
e) gesteigerte L-Methionin-Menge.

### Detaillierte Beschreibung der Erfindung

### a) Allgemeine Begriffe

Als Proteine mit der biologischen Aktivität der S-Adenosylmethionin Synthase", kurz auch metK genannt (E.C.2.5.1.6), werden solche Proteine bezeichnet, die in der Lage sind L-Methionin und ATP zu S-Adenosyl-Methionin umzusetzen. Dem Fachmann sind weitere Details des metK-Proteins bekannt. Die enzymatische Aktivität von metK kann durch Enzymtests nachgewiesen werden, Vorschriften dafür finden sich in: Markham, G.D. et al. (1983) Methods in Enzymology 94:219-222.

Im Rahmen der vorliegenden Erfindung umfassen die Begriffe "L-Methionin", "Methionin", auch die korrespondierenden Salze, wie z. B. Methionin-Hydrochlorid oder Methionin-Sulfat.

"Polynukleotide" bezeichnet im allgemeinen Polyribonukleotide (RNA) und Polydeoxyribonukleotide (DNA), wobei es sich um nicht modifizierte RNA oder DNA oder modifizierte RNA oder DNA handeln kann.

Unter "Polypeptiden" versteht man erfindungsgemäß Peptide oder Proteine, die zwei oder mehr über Peptidbindungen verbundene Aminosäuren enthalten.

Der Begriff "Stoffwechselmetabolit" bezeichnet chemische Verbindungen, die im Stoffwechsel von Organismen als Zwischen- oder auch Endprodukte vorkommen und die neben ihrer Eigenschaft als chemische Bausteine auch modulierende Wirkung auf Enzyme und ihre katalytische Aktivität haben können. Dabei ist aus der Literatur bekannt, dass solche Stoffwechselmetabolite sowohl hemmend als auch stimulierend auf die Aktivität von Enzymen wirken können (Biochemistry, Stryer, Lubert, 1995 W. H. Freeman & Company, New York, New York.). In der Literatur ist auch beschrieben, dass es möglich ist durch Maßnahmen, wie Mutation der genomischen DNA durch UV-Strahlung, ionisierender Strahlung oder mutagene Substanzen und nachfolgender Selektion auf bestimmte Phänotypen, in Organismen solche Enzyme zu produzieren, in denen die Beeinflussung durch Stoffwechselmetabolite verändert wurde (Sahm H., Eggeling L., de Graaf A. A. Biological Chemistry 381 (9-10):899-910, 2000; Eikmanns BJ.. Eggeling L., Sahm H. Antonie van Leeuwenhoek. 64:145-63, 1993-94). Diese veränderten Eigenschaften können auch durch gezielte Maßnahmen erreicht werden. Dabei ist dem Fachmann bekannt, in Genen für Enzyme auch gezielt bestimmte Nukleotide der für das Protein kodierenden DNA so zu verändern, dass das aus der exprimierten DNA-Sequenz resultierende Protein bestimmte neue Eigenschaften aufweist. So kann zum Beispiel erreicht werden, dass die modulierende Wirkung von Stoffwechselmetaboliten gegenüber dem nicht veränderten Protein verändert ist. Enzyme können auch derart in ihrer Aktivität beeinflusst werden, dass es zu einer Verringerung der Reaktionsgeschwindigkeit, oder zu einer Veränderung der Affinität gegenüber dem Substrat kommt.

Die Begriffe "exprimieren" bzw. "Verstärkung" oder Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhten, einen starken Promotor verwenden oder ein Gen verwenden, das für ein entsprechendes Enzym mit einer hohen Aktivität kodiert und man kann gegebenenfalls diese Maßnahmen kombinieren.

Die Begriffe "abschwächen" und "verringern" beschreiben im Kontext der Erfindung die Abschwächung oder Verringerung der intrazellulären Aktivität eines oder mehrerer Enzyme in einem Mikroorganismus, die durch die entsprechende DNA kodiert werden. Dazu kann man beispielsweise ein Gen in einem Organismus deletieren, ein vorhandenes Gen durch ein anderes Gen ersetzen, die Kopienzahl eines Transkriptes des Gens bzw. der Gene emiedrigen, einen schwachen Promotor verwenden oder ein Gen verwenden, das für ein entsprechendes Enzym mit einer niedrigeren Aktivität kodiert und man kann gegebenenfalls diese Maßnahmen kombinieren.

Die "verringerte Aktivität" einer erfindungsgemäßen S-Adenosylmethionin Synthase-Mutante oder eines funktionelle Äquivalents kann durch den Vergleich mit der Aktivität der nativen S-Adenosylmethionin Synthase, wie z.B. aus Corynebacterium glutamicum Wildtyp, ATCC 13032, bestimmt werden. Geeigneterweise bringt man dazu Plasmide, die in Corynebacterium glutamicum replizieren, und die die Gene für S-Adenosylmethionin Synthase-Mutanten tragen, durch Transformation z.B. in Corynebacterium glutamicum Wildtyp, ATCC 13032 ein. Außerdem bringt man entsprechende Plasmide in Corynebacterium glutamicum Wildtyp, ATCC 13032 ein, die das Wildtypenzym S-Adenosylmethionin Synthase exprimieren. Solchermaßen erhaltene Corynebacterium glutamicum Transformanten werden in geeigneten Medien kultiviert und in der logarithmischen Phase des Wachstums bei gleicher OD₆₀₀ geemtet. Danach werden aus den geemteten Zellen beider Transformanten nach bekannten Protokollen Proteinextrakte hergestellt. Gleiche Mengen dieser Proteinextrakte (nach Proteinbestimmung) werden dann in einen S-Adenosylmethionin Synthase Assay nach Markham, G.D. et al. (1983) Methods in Enzymology 94: 219-222 eingesetzt. Die Radioaktivität des gebildeten S-Adenosylmethionins wird in einem Szintillationszähler bestimmt. Unter Berücksichtigung der spezifischen Aktivität des radioaktiven L-Methionins, sowie der eingesetzten Proteinmenge läßt sich die Rate der S-Adenosylmethionin-Bildung aus der Zunahme der eingebauten Radioaktivität pro Zeiteinheit bestimmen. Ihre Einheit lautet µmol S-Adenosylmethionin/min*mg Protein. Diese Rate kann zwischen Wildtypenzym und Mutantenenzym verglichen werden. Nach dem gleichen Prinzip sind ausgehend von anderen Wildtyp-Enzymen mit S-Adenosylmethionin Synthase-Aktivität erfindungsgemäß brauchbare Mutanten herstellbar.

Eine "verringerte Aktivität" erfindungsgemäß ist insbesondere dann gegeben, wenn die spezifische Aktivität der Mutante auf eine Restaktivität von etwa 1 bis 90 %, vorzugsweise 3 bis 70%. wie z.B. 5 bis 10% der Wildtyp-Aktivität verringert ist.

### b) Beichreibene metK-Proteine

Die beschreibenen Polynukleotidsequenzen kodieren für Proteine mit veränderter, insbesondere verringerter S-Adenosylmethionin Synthase Aktivität gemäß obiger Definition.

Vorzugsweise sind die erfindungsgemäß brauchbaren Mutanten durch Substitution eines oder mehrerer konservierter Cysteinreste innerhalb der metK-Aminosäuresequenz grampositiver und/oder gramnegativer, oder insbesondere coryneformer Bakterien zugänglich. Konservierte Cysteinreste sind anhand von Sequenzalignments leicht feststellbar. Als nichtlimitierendes Beispiele für konservierte Cys-Reste in S-Adenosylmethionin Synthasen aus Bakterien sind Cys24 und Cys94 der Enzyms aus C. glutamicum zu nennen, welche in einer Vielzahl von Bakterien zu finden sind.

In einer bevorzugten Gruppe von Mutanten sind Cys24 und oder Cys94 (gemäß metK aus C. glutamicum ATCC 13032) durch eine von Cys verschiedene Aminosäure, vorzugsweise Alanin, substituiert, wodurch die Enzymaktivität in obiger Weise verringert wird.

"Funktionale Äquivalente" oder Analoga der konkret offenbarten Polypeptide sind im Rahmen der vorliegenden Beschreibung davon verschiedene Polypeptide, welche weiterhin die gewünschte biologische Aktivität, wie z.B. Substratspezifität, besitzen.

Unter "funktionalen Äquivalenten" versteht man erfindungsgemäß insbesondere Mutanten, welche in wenigstens einer der oben genannten Sequenzpositionen eine andere als die konkret genannte Aminosäure aufweisen aber trotzdem eine der oben genannten biologische Aktivität besitzen. "Funktionale Äquivalente" umfassen somit die durch eine oder mehrere Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente' umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche ein der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitiemde Beispiele für derartige heterologe Sequenzen sind z.B. Signalpeptide, Enzyme, Immunoglobuline, Oberflächenantigene, Rezeptoren oder Rezeptorliganden.

Erfindungsgemäß mit umfasste "funktionale Äquivalente" sind Homologe zu den konkret offenbarten Proteinen. Diese besitzen wenigstens 20%, 30%. oder etwa 40%. 50 %. vorzugsweise wenigstens etwa 60 %, 65%, 70%, oder 75% insbesondere wenigstens 85 %, wie z.B. 90%, 95% oder 99%, Homologie zu einer der konkret offenbarten Sequenzen, berechnet nach dem Algorithmus von Pearson und Lipman, Proc. Natl. Acad, Sci. (USA) 85(8), 1988, 2444-2448.

Mutanten und funktionale Analoga enthalten die charakteristische Teilsequenz

G(FIY)(D/S)X¹X²(S/T)X³(G/A)V

gemäß obiger Definition ; wobei X³ eine von Cys verschiedene, durch Mutation eingeführte Aminosäure, insbesondere Alanin, darstellt. X³ entspricht Cys94 der metK Wildtyp-Sequenz von C. glutamicum (SEQ ID NO:16). X² steht vorzugsweise für Ala, Glu, Asp, Asn oder Arg; und X¹ steht vorzugsweise für Gly, Cys, Ser oder Ala.

Homologe der beschreibenen Proteine oder Polypeptide können durch Mutagenese erzeugt werden, zB. durch Punktmutation oder Verkürzung des Proteins. Der Begriff "Homolog", wie er hier verwendet wird, betrifft eine variante Form des Proteins, die als Agonist oder Antagonist der Protein-Aktivität wirkt.

Homologe des beschreibene Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen codieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:4477).

Zusätzlich können Banken von Fragmenten des Protein-Codons verwendet werden, um eine variegierte Population von Protein-Fragmenten zum Screening und zur anschließenden Selektion von Homologen eines erfindungsgemäßen Proteins zu erzeugen. Bei einer Ausführungsform kann eine Bank von kodierenden Sequenzfragmenten durch Behandeln eines doppelsträngigen PCR-Fragmentes einer kodierenden Sequenz mit einer Nuklease unter Bedingungen, unter denen ein Nicking nur etwa einmal pro Molekül erfolgt, Denaturieren der doppelsträngigen DNA, Renaturieren der DNA unter Bildung doppelsträngiger DNA, die Sense-/Antisense-Paare von verschiedenen genickten Produkten umfassen kann, Entfernen einzelsträngiger Abschnitte aus neu gebildeten Duplices durch Behandlung mit S1-Nudease und Ligieren der resultierenden Fragmentbank in einen Expressionsvektor erzeugt werden. Durch dieses Verfahren kann eine Expressionsbank hergeleitet werden, die N-terminate, C-terminale und interne Fragmente mit verschiedenen Größen des erfindungsgemäßen Proteins kodiert.

Im Stand der Technik sind mehrere Techniken zur Mutagenese von Genen bekannt:coco, WM et al. 2001. DNA shuffling method for generating highly recombined genes and evolved enzymes. Nature Biotechnol. 19:354-359; DE 19953854; Leung DW et al. 1989. A method for random mutagenesis of a defined DNA segment using a modified polymerase chain reaction. Technique 1:11-15; Stemmer WPC 1994. DNA shuffling by random fragmentation and reassembly: in vitro recombination for molecular evolution. Proc. Natl. Acad. Sci USA 91:10747-10751; und US 5811238. Diese Verfahren sind zur Herstellung erfindungsgemäß brauchbarer Mutanten einsetzbar.

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese von erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen codiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331.

### c) Beschreibenen Polynukleotide

Gegenstand der Beschreibung sind ebenso Nukleinsäuresequenzen (einzel- und doppelsträngige DNA- und RNA-Sequenzen, wie z.B. cDNA und mRNA), kodierend für ein metK-Enzym und deren funktionalen äquivalenten, welche z.B. auch unter Verwendung künstlicher Nukleotidanaloga zugänglich sind.

Die Beschreibung betrifft sowohl isolierte Nukleinsäuremoleküle, welche für die genannten Polypeptide bzw. Proteine oder biologisch aktive Abschnitte davon kodieren, sowie Nukleinsäurefragmente, die z.B. als Hybridisierungssonden oder Primer zur Identifizierung oder Amplifizierung von erfindungsgemäßer kodierenden Nukleinsäuren verwendet werden können.

Die beschriebenen Nukleinsäuremoleküle können zudem untranslatierte Sequenzen vom 3'- und/oder 5'-Ende des kodierenden Genbereichs enthalten

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Die Beschreibung umfasst weiterhin die zu den konkret beschriebenen Nukleotidsequenzen komplementären Nukleinsäuremoleküle oder einen Abschnitt davon.

Die beschriebenen Nukleotidsequenzen ermöglichen die Erzeugung von Sonden und Primem, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter stringenten Bedingungen an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert. Weitere beschriebene, Nukleinsäuresequenzen sind abgeleitet von SEQ ID NO:21 und unterscheiden sich davon durch Addition, Substitution, Insertion oder Deletion einzelner oder mehrerer Nukleotide, kodieren aber weiterhin für Polypeptide mit dem gewünschten Eigenschaftsprofil. Dies können Polynukleotide sein. die zu obigen Sequenzen in mindestens etwa 50%, 55%**,** 60%, 65%**,** 70%, 80% oder 90%, vorzugsweise in mindestens etwa 95%, 96%, 97%, 98% oder 99% der Sequenzpositionen identisch sind.

die Beschreibung umfasst sind auch solche Nukleinsäuresequenzen, die sogenannte stumme Mutationen umfassen oder entsprechend der Codon-Nutzung eines speziellen Ursprungs- oder Wirtsorganismus, im Vergleich zu einer konkret genannten Sequenz verändert sind, ebenso wie natürlich vorkommende Varianten, wie z.B. Allelvarianten, davon. Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Beschreibung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Weiterhin umfasst die Beschreibung auch Nukleinsäuresequenzen, welchen mit oben genannten kodierenden Sequenzen hybridisieren oder dazu komplementär sind. Diese Polynukleotide lassen sich bei Durchmusterung von genomischen oder cDNA-Banken auffinden und gegebenenfalls daraus mit geeigneten Primem mittels PCR vermehren und anschließend beispielsweise mit geeigneten Sonden isolieren. Eine weitere Möglichkeit bietet die Transformation geeigneter Mikroorganismen mit den Polynukleotiden oder Vektoren, die Vermehrung der Mikroorganismen und damit der Polynukleotide und deren anschließende Isolierung. Darüber hinaus können die Polynukleotide auch auf chemischem Wege synthetisiert werden.

Unter der Eigenschaft, an Polynukleotide "hybridisieren" zu können, versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northem- oder Southem-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northem-Blot-Technik die Verwendung einer 50 - 70°C, vorzugsweise 60 - 65 °C warmen Waschlösung, beispielsweise 0.1x SSC-Puffer mit 0,1 % SDS (20x SSC: 3M NaCl. 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z:B. in Ausubel et al., Current Protocols in Molecular Biology, John Wiley & Sons, N.Y. (1989), 6.3.1-6.3.6. beschrieben.

### d) Isolierung der kodierenden metK-Gene und anderer Gene

Die für das Enzym S-Adenosylmethionin-Synthase (EC 2.5.1.6) kodierenden metK Gene sind in an sich bekannter Weise isolierbar.

Zur Isolierung der metK-Gene oder auch anderer Gene anderer Organismen wird zunächst eine Genbank dieses Organsimus in Escherichia coli (E. coli) angelegt. Das Anlegen von Genbanken ist in allgemein bekannten Lehrbüchern und Handbüchem ausführlich beschrieben. Als Beispiel seien das Lehrbuch von Winnacker: Gene und Klone, Eine Einführung in die Gentechnologie (Verlag Chemie, Weinheim. Deutschland, 1990), oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495-508 (198)) in λ-Vektoren angelegt wurde.

Zur Herstellung einer Genbank in E. coli können Cosmide, wie der Cosmidvektor SuperCos (Wahl et al. (1987), Proceedings of the National Academy of Sciences USA 84: 2160-2164), aber auch Plasmide, wie pBR322 (Bolivar, Life Sciences. 25. 807-818 (1979)) oder pUC9 (Vieira et al., 1982, Gene, 19: 259-268), verwendet werden. Als Wirte eignen sich besonders solche E. coli Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde. Die mit Hilfe von Cosmiden klonierten langen DNA-Fragmente können anschließend wiederum in gängige, für die Sequenzierung geeignete Vektoren subkloniert und anschließend sequenziert werden, so wie es z. B. bei Sanger et al. (Proceedings of the National Academy of Sciences of the United States of America, 74: 5463-5467, 1977) beschrieben ist

Die erhaltenen DNA-Sequenzen können dann mit bekannten Algorithmen bzw. Sequenzanalyse-Programmen, wie z. B. dem von Staden (Nucleic Acids Research (1986) 14,217-232), dem von Marck (Nucleic Acids Research (1988) 16, 1829-1836) oder dem GCG-Programm von Butler (Methods of Biochemical Analysis (1998) 39, 74-97), untersucht werden.

Anleitungen zur Identifizierung von DNA-Sequenzen mittels Hybridisierung findet der Fachmann unter anderem im Handbuch The DIG System Users Guide für Filter Hybridization" der Firma Boehringer Mannheim GmbH (Mannheim, Deutschland, 1993) und bei Liebl et al. (International Journal of Systematic Bacteriology (1991) 41: 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: A Practical Approach (IRL Press, Oxford, UK, 1984) und bei Newton und Graham: PCR (Spektrum Akademischer Verlag, Heidelberg, Deutschland, 1994).

Weiterhin ist bekannt, dass Änderungen am N- und/oder C- Terminus eines Proteins dessen Funktion nicht wesentlich beeinträchtigen oder sogar stabilisieren können. Angaben hierzu findet der Fachmann unter anderem bei Ben-Bassat et al. (1987) Journal of Bacteriology 169: 751-757, bei O'Regan et al. (1989) Gene 77: 237-251, bei Sahin-Toth et al. (1994) Protein Sciences 3: 240-247, bei Hochuli et al. (1988) Biotechnology 6: 1321-1325 und in bekannten Lehrbüchern der Genetik und Molekularbiologie.

### e) Verwendete Wirtszellen

Für das erfindungsgemäße Verfahren verwendet man vorzugsweise coryneforme Bakterien, deren verringerte metK-Aktivität über wenigstens eine der folgenden Eigenschaften nachweisbar ist:
a) einen im Vergleich zum Wildtyp-Stamm geringeren intrazellulären S-Adenosylmethionin Titer;
b) eine geringere intrazelluläre S-Adenosylmethionin Synthase Konzentration (weniger S-Adenosylmethionin Synthase bezogen auf Gesamtprotein); oder
c) eine geringere intrazelluläre S-Adenosylmethionin Synthase Aktivität (weniger S-Adenosylmethionin Synthase Enzymaktivität bezogen auf S-Adenosylmethionin Synthase-Proteingehalt.

Sämtliche dieser Eigenschaften sind in einfacher Weise vom Fachmann, gegebenenfalls unter Heranziehung der vorliegenden Beschreibung, bestimmbar.

Weitere Gegenstände der Beschreibung betreffen insbesondere als Wirtszelle dienende Mikroorganismen, insbesondere coryneforme Bakterien, die einen Vektor, insbesondere Pendelvektor oder Plasmidvektor, der wenigstens ein metK Gen erfindungsgemäßer Definition trägt, enthalten oder in denen ein erfindungsgemäßes metK Gen mit verringerter Aktivität exprimiert ist.

Diese Mikroorganismen können schwefelhaltige Feinchemikalien, L-Methionin, aus Glucose, Saccharose, Lactose, Fructose, Maltose, Melasse, Stärke, Cellulose oder aus Glycerin und Ethanol herstellen. Es sind dies coryneforme Bakterien, insbesondere der Gattung Corynebacterium. Aus der Gattung Corynebacterium ist insbesondere die Art Corynebacterium glutamicum zu nennen, die in der Fachwelt für ihre Fähigkeit bekannt ist, L-Aminosäuren zu produzieren.

Als Beispiele für geeignete Stämme coryneformer Bakterien sind solche der Gattung Corynebacterium, insbesondere der Art Corynebacterium glutamicum (C. glutamicum), wie
Corynebacterium glutamicum ATCC 13032,
Corynebacterium acetoglutamicum ATCC 15806,
Corynebacterium acetoacidophilum ATCC 13870,
Corynebacterium thermoaminogenes FERM Bp-1539,
Corynebacterium melassecola ATCC 17965
oder
der Gattung Brevibacterium, wie
Brevibacterium flavum ATCC 14067
Brevibacterium lactofermentum ATCC 13869 und
Brevibacterium divaricatum ATCC 14020 zu nennen;
oder davon abgeleitete Stämme, wie
Corynebacterium glutamicum KFCC10065
Corynebacterium glutamicum ATCC21608
welche ebenfalls L- Methionin oder deren Vorstufe(n) produzieren, aufgeführt.( KFCC = Korean Federation of Culture Collection; ATCC = American Type Culture Collection)

### f) Durchführung der erfindungsgemäßen Fermentation

Erfindungsgemäß wurde festgestellt, dass coryneforme Bakterien nach Expression eines erfindungsgemäßen metK Gens in vorteilhafter Weise L-Methionin, produzieren.

Um die Aktivität oder Menge eines Enzyms, z.B. der S-Adenosylmethionin Synthase, metK, zu verringern, kann der Fachmann unterschiedliche Maßnahmen einzeln oder in Kombinationen ausführen. Durch Reduktion der Transkriptionshäufigkeit des Gens, das für das erfindungsgemäße Protein kodiert, kann die Konzentration des betreffenden Proteins gesenkt werden. Dies kann der Fachmann durch Veränderung oder Austausch der Promotor- oder Regulationsregion, sowie der Ribosomenbindungsstelle des kodierenden Gens erreichen. Stromab der kodierenden Region kann der Fachmann Terminatoren verändern oder Sequenzen einfügen, die zu einer verringerten Stabilität des Transkriptes führen. Diese, die Lebensdauer der mRNA verringernden Maßnahmen ermöglichen, die Expression des zugehörigen Proteins, und damit seine Konzentration abzusenken.

Auf der Ebene des exprimierten Enzyms können fusionierte Sequenzen zu einer erhöhten Abbaurate und damit ebenfalls zu einer Absenkung der Konzentration des Proteins führen. Außerdem kann der Fachmann durch gezielte oder ungerichtete Mutagenese des kodierenden Gens die Aktivität, die Substrataffinität und die Substratspezifität verändern. Enzyme können durch Mutationen in den korrespondierenden Genen derart in ihrer Aktivität beeinflußt werden, dass es zu einer teihmeisen oder vollständigen Verringerung der Reaktionsgeschwindigkeit der enzymatischen Reaktion kommt. Beispiele für solche Mutationen sind dem Fachmann bekannt (Motoyama H. Yano H. Terasaki Y. Anazawa H. Applied & Environmental Microbiology. 67:3064-70, 2001, Eikmanns BJ. Eggeling L. Sahm H. Antonie van Leeuwenhoek. 64:145-63, 1993-94) Mutanten des Proteins können auch zu verringerter oder verhinderter Homo- oder Heteromultimerisierung von Enzymkomplexen und damit ebenfalls zu einer Verschlechterung der enzymatischen Eigenschaften führen.

Solchermaßen veränderte Gene können entweder in Plasmiden oder bevorzugt im Chromosom integriert vorliegen. Dabei kann das ursprüngliche, nicht auf diese Art veränderte Gen noch zusätzlich vorhanden sein, bevorzugt aber gegen das veränderte Gen ausgetauscht sein.

Um die Aktivität eines Enzyms, z.B. der S-Adenosylmethionin Synthase (metK), gemessen in einem coryneformen Bakterium, zu verringern, kann es ausreichend sein, Gene, die für funktionale Äquivalente, wie künstlich hergestellte Mutanten oder natürliche Homologe aus anderen Organsimen kodieren, zu exprimieren. Dabei kann das ursprüngliche Gen noch zusätzlich vorhanden sein, bevorzugt aber gegen das veränderte oder homologe Gen ausgetauscht sein.

Zusätzlich kann es für die Produktion von L-Methionin_{µ} durch Fermentation in coryneformen Bakterien, vorteilhaft sein, neben einer Expression eines erfindungsgemäßen metK-Gen eines oder mehrere Enzyme des jeweiligen Biosyntheseweges, des Cystein-Stoffwechselwegs, der Aspartatsemialdehyd-Synthese, der Glykolyse, der Anaplerotik, des Pentose-Phosphat-Stoffwechsels, des Zitronensäure-Zyklus oder des Aminosäure-Exports zu verstärken.

So kann für die Herstellung von schwefelhaltige Feinchemikalien, insbesondere L-Methionin, eines oder mehrere der folgenden Gene verstärkt sein:
- das für eine Aspartatkinase kodierende Gen lysC (EP 1 108 790 A2; DNA-SEQ NO. 281),
- das für eine Aspartat-Semialdehyd kodierende Gen asd (EP 1 108 790 A2; DNA-SEQ NO. 282),
- das für die Glycerinaldehyd-3-Phosphat Dehydrogenase kodierende Gen gap (Eikmanns (1992), Journal of Bacteriology 174: 6076-6086),
- das für die 3-Phosphoglycerat Kinase kodierende Gen pgk (Eikmanns (1992), Journal of Bacteriology 174: 6076-6086),
- das für die Pyruvat Carboxylase kodierende Gen pyc (Eikmanns (1992), Journal of Bacteriology 174: 6076-6086),
- das für die Triosephosphat Isomerase kodierende Gen tpi (Eikmanns (1992), Journal of Bacteriology 174: 6076-6086),
- das für die Homoserin O-Acetyltransferase kodierende Gen metA (EP 1 108 790 A2; DNA-SEQ NO. 725),
- das für die Cystathionin-gamma-Synthase kodierende Gen metB (EP 1 108 790 A2; DNA-SEQ NO. 3491),
- das für die Cystathionin-gamma-Lyase kodierende Gen metC (EP 1 108 790 A2; DNA-SEQ NO. 3061),
- das für die Cystathionin-Synthase kodierende Gen metH (EP 1 108 790 A2; DNA-SEQ NO. 1663),
- das für die Serin-Hydroxymethyltransferase kodierende Gen glyA (EP 1 108 790 A2; DNA-SEQ NO. 1110),
- das für die O-Acetylhomoserin-Sulfhydrylase kodierende Gen metY (EP 1 108 790 A2; DNA-SEQ NO. 726),
- das für die Methylentetrahydrofolat-Reduktase kodierende Gen metF (EP 1 108 790 A2; DNA-SEQ NO. 2379),
- das für die Phosphoserin-Aminotransferase kodierende Gen serC (EP 1 108 790 A2; DNA-SEQ NO. 928)
- eines für die Phosphoserin-Phosphatase kodierende Gen serB (EP 1 108 790 A2; DNA-SEQ NO. 334, DNA-SEQ NO. 467**,** DNA-SEQ NO. 2767)
- das für die Serine Acetyl-Transferase kodierende Gen cysE (EP 1 108 790 A2; DNA-SEQ NO. 2818)
- das für die Cystein-Synthase kodierende Gen cysK (EP 1 108 790 A2; DNA-SEQ NO. 2817),
- das für eine Homoserin-Dehydrogenase kodierende Gen hom (EP 1 108 790 A2; DNA-SEQ NO. 1306)

So kann für die Herstellung von L-Methionin in coryneformen Bakterien, vorteilhaft sein, gleichzeitig wenigstens eines der nachfolgenden Gene zu mutieren, so dass die korrespondierenden Proteine, verglichen mit nicht mutierten Proteinen, in geringerem Maße oder nicht durch einen Stoffwechselmetaboliten in ihrer Aktivität beeinflusst werden oder so dass ihre spezifische Aktivität gesteigert wird:
- das für eine Aspartatkinase kodierende Gen lysC (EP 1 108 790 A2; DNA-SEQ NO. 281),
- das für die Pyruvat Carboxylase kodierende Gen pyc (Eikmanns (1992), Journal of Bacteriology 174: 6076-6086),
- das für die Homoserin O-Acetyltransferase kodierende Gen metA (EP 1 108 790 A2; DNA-SEQ NO. 725),
- das für die Cystathionin-gamma-Synthase kodierende Gen metB (EP 1 108 790 A2; DNA-SEQ NO. 3491),
- das für die Cystathionin-gamma-Lyase kodierende Gen metC (EP 1 108 790 A2; DNA-SEQ NO. 3061),
- das für die Methionin-Synthase kodierende Gen metH (EP 1 108 790 A2; DNA-SEQ NO. 1663),
- das für die Serin-Hydroxymethyltransferase kodierende Gen glyA (EP 1 108 790 A2; DNA-SEQ NO. 1110),
- das für die O-Acetylhomoserin-Sulfhydrylase kodierende Gen metY (EP 1 108 790 A2; DNA-SEQ NO. 726),
- das für die Methylentetrahydrofolat-Reduktase kodierende Gen metF (EP 1 108 790 A2; DNA-SEQ NO. 2379),
- das für die Phosphoserin-Aminotransferase kodierende Gen serC (EP 1 108 790 A2; DNA-SEQ NO. 928)
- eines für die Phosphoserin-Phosphatase kodierende Gen serB (EP 1 108 790 A2; DNA-SEQ NO. 334, DNA-SEQ NO. 467, DNA-SEQ NO. 2767)
- das für die Serine Acetyl-Transferase kodierende Gen cysE (EP 1 108 790 A2; DNA-SEQ NO. 2818)
- das für die Cystein-Synthase kodierende Gen cysK (EP 1 108 790 A2; DNA-SEQ NO. 2817),
- das für eine Homoserin-Dehydrogenase kodierende Gen hom (EP 1 108 790 A2; DNA-SEQ NO. 1306)

Weiterhin kann es für die Produktion von L-Methionin vorteilhaft sein, zusätzlich zur Expression eines der erfindungsgemäßen metK-Gene eines oder mehrere der folgenden Gene abzuschwächen, insbesondere deren Expression zu verringern, oder auszuschalten:
- das für die Homoserine-Kinase kodierende Gen thrB (EP 1 108 790 A2; DNA-SEQ NO. 3453)
- das für die Threonin Dehydratase kodierende Gen ilvA (EP 1 108 790 A2; DNA-SEQ NO. 2328)
- das für die Threonin Synthase kodierende Gen thrC (EP 1 108 790 A2; DNA-SEQ NO. 3486)
- das für die Meso-Diaminopimelat D-Dehydrogenase kodierende Gen ddh (EP 1 108 790 A2; DNA-SEQ NO. 3494)
- das für die Phosphoenolpyruvat-Carboxykinase kodierende Gen pck (EP 1 108 790 A2; DNA-SEQ NO. 3157)
- das für die Glucose-6-Phosphat-6-Isomerase kodierende Gen pgi (EP 1 108 790 A2; DNA-SEQ NO. 950)
- das für die Pyruvat-Oxidase kodierende Gen poxB (EP 1 108 790 A2; DNA-SEQ NO. 2873)
- das für die Dihydrodipicolinat Synthase kodiernde Gen dapA(EP 1 108 790 A2; DNA-SEQ NO. 3476)
- das für die Dihydrodipicolinat Reduktase kodiernde Gen dapB (EP 1 108 790 A2; DNA-SEQ NO. 3477)
- das für die Diaminopicolinat Decarboxylase kodiemde Gen lysA (EP 1 108 790 A2; DNA-SEQ NO. 3451)

Weiterhin kann es für die Produktion von L-Methionin vorteilhaft sein, zusätzlich zur Expression eines der erfindungsgemäßen metK-Gene in coryneformen Bakterien gleichzeitig wenigstens eines der folgenden Gene so zu mutieren, dass die enzymatische Aktivität des korrespondierenden Proteins teilweise oder vollständig verringert wird:
- das für die Homoserine-Kinase kodierende Gen thrB (EP 1 108 790 A2; DNA-SEQ NO. 3453)
- das für die Threonin Dehydratase kodierende Gen ilvA (EP 1 108 790 A2; DNA-SEQ NO. 2328)
- das für die Threonin Synthase kodierende Gen thrC (EP 1 108 790 A2; DNA-SEQ NO. 3486)
- das für die Meso-Diaminopimelat D-Dehydrogenase kodierende Gen ddh (EP 1 108 790 A2; DNA-SEQ NO. 3494)
- das für die Phosphoenolpyruvat-Carboxykinase kodierende Gen pck (EP 1 108. 790 A2; DNA-SEQ NO. 3157)
- das für die Glucose-6-Phosphat-6-Isomerase kodierende Gen pgi (EP 1 108 790 A2; DNA-SEQ NO. 950)
- das für die Pyruvat-Oxidase kodierende Gen poxB (EP 1 108 790 A2; DNA-SEQ NO. 2873)
- das für die Dihydrodipicolinat Synthase kodiemde Gen dapA(EP 1 108 790 A2; DNA-SEQ NO. 3476)
- das für die Dihydrodipicolinat Reduktase kodiemde Gen dapB (EP 1 108 790 A2; DNA-SEQ NO. 3477)
- das für die Diaminopicolinat Decarboxylase kodiernde Gen lysA (EP 1 108 790 A2; DNA-SEQ NO. 3451)

Weiterhin kann es für die Produktion von L-Methioninµ vorteilhaft sein, neben der Expression eines erfindungsgemäßen metK-Gens unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London. UK, 1982).

Zur Erzielung einer Überexpression kann der Fachmann unterschiedliche Maßnahmen einzeln oder in Kombination ergreifen. So kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Methionin-Produktion zu steigern. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Biotechnology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der EP 0472869, in US 4,601,893, bei Schwarzer und Pühler (Biotechnology 9, 84-87 (1991), bei Remscheid et al. (Applied and Environmental Microbiology 60,126-132 (1994), bei LaBarre et al. (Journal of Bacteriology 175, 1001-1007 (1993)), in der WO 96/15246, bei Malumbres et al. (Gene 134, 15-24 (1993)), in der JP-A-10-229891, bei Jensen und Hammer (Biotechnology and Bioengineering 58,.191-195 (1998)), bei Makrides (Microbiological Reviews 60:512-538 (1996) und in bekannten Lehrbüchem der Genetik und Molekularbiologie.

Gegenstand der Beschreibung sind deshalb auch Expressionskonstrukte, enthaltend unter der genetischen Kontrolle regulativer Nukleinsäuresequenzen für ein genanntes, Polypeptid kodierende Nukleinsäuresequenz; sowie Vektoren, umfassend wenigstens eines dieser Expressionskonstrukte. Vorzugsweise umfassen solche Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Aktivierungssequenzen sowie Enhancer und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).
- Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt es werden keine zusätzlichen Regulationssignale vor das Strukturgen insertiert und der natürliche Promotor mit seiner Regulation wird nicht entfernt. Statt dessen wird die natürliche Regulationssequenz so mutiert, dass keine Regulation mehr erfolgt und die Genexpression gesteigert oder verringert wird. Die Nukleinsäuresequenzen können in einer oder mehreren Kopien im Genkonstrukt enthalten sein.

Beispiele für brauchbare Promotoren sind: die Promotoren, ddh, amy, lysC, dapA, lysA aus Corynebacterium glutamicum, aber auch gram-positive Promotoren, wie SPO2 wie sie in Bacillus Subtilis and Its Closest Relatives, Sonenshein, Abraham L.,Hoch, James A., Losick, Richard; ASM Press, District of Columbia, Washington und Patek M. Eikmanns BJ. Patek J. Sahm H. Microbiology. 142 1297-309, 1996 beschrieben sind, oder aber auch cos-, tac-, trp-, tet-, trp-tet-, Ipp-, lac-, Ipp-lac-, laclq-, T7-, T5-. T3-, gal-, trc-, ara-, SP6-, I-PR oder I-PL-Promotor, die vorteilhafterweise in gram-negativen Bakterien Anwendung finden. Bevorzugt ist auch die Verwendung induzierbarer Promotoren, wie z.B. licht- und insbesondere temperaturinduztierbarer Promotoren, wie der PᵣPᵣ-Promotor. Prinzipiell können alle natürlichen Promotoren mit ihren Regulationssequenzen verwendet werden. Darüber hinaus können auch synthetische Promotoren vorteilhaft verwendet werden.

Die genannten regulatorischen Sequenzen sollen die gezielte Expression der Nukleinsäuresequenzen ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Expression negativ beeinflussen und dadurch verringem. So kann eine Abschwächung auf der Transkriptionsebene erfolgen, indem schwache Transkriptionssignale wie Promotoren und/oder "Enhancer" verwendet werden. Daneben ist aber auch eine Abschwächung der Translation möglich, indem beispielsweise die Stabilität der mRNA verringert wird.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Die Herstellung einer Expressionskassette erfolgt durch Fusion eines geeigneten Promotors, einer geeigneten Shine-Dalgarno-Sequenz mit einer metK-Nukleotidsequenz sowie einem geeigneten Terminationssignal. Dazu verwendet man gängige Rekombinations- und Klonierungstechniken, wie sie beispielsweise in Current Protocols in Molecular Biology, 1993, John Wiley & Sons, Incorporated, New York New York, PCR Methods, Gelfand, David H., Innis, Michael A., Sinsky, John J. 1999, Academic Press, Incorporated, Califomia, San Diego, ., PCR Cloning Protocols, Methods in Molecular Biology Ser., Vol. 192, 2nd ed., Humana Press, New Jersey, Totowa. T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Die beschriebenen metK Gene werden beispielhaft mit Hilfe von episomalen Plasmiden exprimiert. Als Plasmide eignen sich solche, die in coryneformen Bakterien repliziert werden. Zahlreiche bekannte Plasmidvektoren, wie z. B. pZ1 (Menkel et al., Applied and Environmental Microbiology (1989) 64: 549-554), pEKEx1 (Eikmanns et al., Gene 102: 93-98 (1991)) oder pHS2-1 (Sonnen et al., Gene 107: 69-74 (1991)) beruhen auf den kryptischen Plasmiden pHM1519, pBL1 oder pGA1. Andere Plasmidvektoren, wie z. B. pCLiK5MCS, SEQ ID NO:9, oder solche, die auf pCG4 (US-A 4,489,160) oder pNG2 (Serwold-Davis et al., FEMS Microbiology Letters 66, 119-124 (1990)) oder pAG1 (US-A 5,158.891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Remscheid et al. (Applied and Environmental Microbiology 60,126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise pSUP301 (Simon et al., Bio/ Technology 1,784-791 (1983)), pK18mob oder pK19mob (Schäfer et al., Gene 145,69-73 (1994)), Bemard et al., Journal ofMolecular Biology, 234: 534-541 (1993)), pEM1 (Schrumpf et al. 1991, Journal of Bacteriology 173: 4510-4516) oder pBGS8 (Spratt et al.,1986, Gene 41: 337-342) in Frage. Andere Plasmidvektoren, wie z. B. pCLiK5MCS integrativ sacB, SEQ ID NO:12, können in gleicher Weise verwendet werden.

Der Plasmidvektor, der das zu amplifizierende Gen enthält, wird anschließend durch Transformation in den gewünschten Stamm von C. glutamicum überführt Methoden zur Transformation sind beispielsweise bei Thierbach et al. (Applied Microbiology and Biotechnology 29, 356-362 (1988)), Dunican und Shivnan (Biotechnology 7, 1067-1070 (1989)) und Tauch et al. (FEMS Microbiological Letters 123,343-347 (1994)) beschrieben.

Die Mikroorganismen können kontinuierlich oder diskontinuierlich im batch Verfahren (Satzkultivierung) oder im fed batch (Zulaufverfahren) oder repeated fed batch Verfahren (repetitives Zulaufverfahren) zur Produktion von schwefelhaltige Feinchemikalien, insbesondere L-Methionin, kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeektrakt, Fleischextrakt und andere. Die Stickstoffquellen können einzeln oder als Mischung verwendet werden.

Anorganische Salzverbindungen, die in den Medien enthalten sein können, umfassen die Chlorid-,Phosphor- oder Sulfatsalze von Calcium, Magnesium, Natrium, Kobalt, Molybdän, Kalium, Mangan, Zink, Kupfer und Eisen.

Als Schwefelquelle für die Herstellung von Methioninµ können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach° (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121 °C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht läßt sich während der Anzucht durch Zugabe von basischen Verbindungen wie Natriumhydroxid, Kaliumhydroxid, Ammoniak bzw. Ammoniakwasser oder sauren Verbindungen wie Phosphorsäure oder Schwefelsäure kontrollieren. Zur Kontrolle der Schaumentwicklung können Antischaummittel wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuefalten, werden Sauerstoff oder Sauerstoff haltige Gasmischungen, wie z. B. Umgebungsluft, in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen L-Methionin enthaltendenµ Fermentationsbrühen haben üblicherweise eine Trockenmasse von 7,5 bis 25 Gew.-%.

Vorteilhaft ist außerdem auch, wenn die Fermentation zumindest am Ende, insbesondere jedoch über mindestens 30% der Fermentationsdauer zuckerlimitiert gefahren wird. Das heißt, dass während dieser Zeit die Konzentration an verwertbarem Zucker im Fermentationsmedium auf ≥ 0 bis 3 g/l gehalten, beziehungsweise abgesenkt wird.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Anschließend kann die Fermentationsbrühe mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Diese aufkonzentrierte Fermentationsbrühe kann anschließend durch Gefriertrocknung, Sprühtrocknung, Sprühgranulation oder durch anderweitige Verfahren aufgearbeitet werden.

Es ist aber auch möglich, die L-Methionin_{µ} weiter aufzureinigen. Hierzu wird die produkthaltige Brühe nach dem Abtrennen der Biomasse einer Chromatographie mit einem geeigneten Harz unterworfen, wobei das gewünschte Produkt oder die Verunreinigungen ganz oder teilweise auf dem Chromatographieharz zurückgehalten werden. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung bewandert. Das gereinigte Produkt kann durch Filtration oder Ultrafiltration konzentriert und bei einer Temperatur aufbewahrt werden, bei der die Stabilität des Produktes maximal ist.

Die Identität und Reinheit der isolierten Verbindung(en) kann durch Techniken des Standes der Technik bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefaßt in: Patek et al. (1994) Appl. Environ. Microbiol. 60:133-140; Malakhova et al. (1996) Biotekhnologiya 11 27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

Die Erfindung wird nun anhand der folgenden nicht-limitierenden Beispiele näher beschrieben:

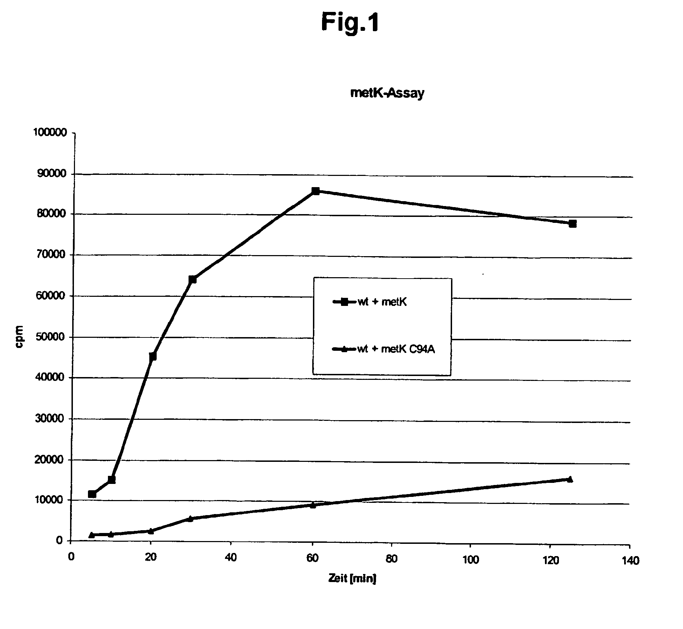

Figur 1 zeigt die Ergebnisse eines radioaktiven metK Assays, durchgeführt mit Wildtypenzym bzw. C94A Mutante.

### Beispiel 1

### Herstellung des Vektors pCLiK5MCS

Zunächst wurden Ampicillinresistenz und Replikationsursprung des Vektors pBR322 mit den Oligonukleotidprimern SEQ ID NO:1 und SEQ ID NO:2 mit Hilfe der Polymerase-Kettenreaktion (PCR) amplifiziert.
SEQ ID NO:1
   5'-CCCGGGATCCGCTAGCGGCGCGCCGGCCGGCCCGGTGTGAAATACCGCACAG-3'
SEQ ID NO:2
   5'-TCTAGACTCGAGCGGCCGCGGCCGGCCTTTAAATTGAAGACGAAAGGGCCTCG-3'

Neben den zu pBR322 komplementären Sequenzen, enthält der Oligonukleotidprimer SEQ ID NO:1 in 5'-3' Richtung die Schnittstellen für die Restriktionsendonukleasen Smal, BamHI, Nhel und Ascl und der Oligonukleotidprimer SEQ ID NO:2 in 5'-3' Richtung die Schnittstellen für die Restriktionsendonukleasen Xbal, Xhol, Notl und Dral. Die PCR Reaktion wurde nach Standardmethode von Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 2,1 kb wurde mit dem GFX™PCR, DNA and Gel Band Purification Kit (Amersham Pharmacia, Freiburg) nach Angaben des Herstellers gereinigt. Die stumpfen Enden des DNA-Fragmentes wurden mit dem Rapid DNA Ligation Kit (Roche Diagnostics, Mannheim) nach Angaben des Herstellers miteinander ligiert und der Ligationsansatz nach Standardmethoden wie in Sambrook et al. (Molecular Cloning. A Laboratory Manual, Cold Spring Harbor, beschrieben (1989)), in kompetente E.coli XL-1Blue (Stratagene, La Jolla, USA) transformiert. Eine Selektion auf Plasmid tragende Zellen wurde durch das Ausplattieren auf Ampicillin (50µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK1.

Ausgehend vom Plasmid pWLT1 (Liebl et al., 1992) als Template für eine PCR Reaktion wurde mit den Oligonukleotidprimern SEQ ID NO:3 und SEQ ID NO:4 eine Kanamycin-Resistenzcassette amplifiziert.
SEQ ID NO:3:
   5'-GAGATCTAGACCCGGGGATCCGCTAGCGGGCTGCTAAAGGAAGCGGA-3'
SEQ ID NO:4:
   5'-GAGAGGCGCGCCGCTAGCGTGGGCGAAGAACTCCAGCA-3'

Neben den zu pWLT1 komplementären Sequenzen, enthält der Oligonukleotidprimer SEQ ID NO:3 in 5'-3' Richtung die Schnittstellen für die Restriktionsendonukleasen Xbal, Smal, BamHl, Nhel und der Oligonukleotidprimer SEQ ID NO:4 in 5'-3' Richtung die Schnittstellen für die Restriktionsendonukleasen Ascl und Nhel. Die PCR Reaktion wurde nach Standardmethode von Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 1,3 kb wurde mit dem GFX™PCR, DNA and Gel Band Purification Kit (Amersham Pharmacia, Freiburg) nach Angaben des Herstellers gereinigt. Das DNA-Fragment wurde mit den Restriktionsendonukleasen Xbal und Ascl (New England Biolabs, Beverly, USA) geschnitten und im Anschluß daran erneut mit dem GFX™PCR, DNA and Gel Band Purification Kit (Amersham Pharmacia, Freiburg) nach Angaben des Herstellers gereinigt. Der Vektor pCLiK1 wurde ebenfalls mit den Restriktionsendonukleasen Xbal und Ascl geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 2,1kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Ampicillin (50µg/ml) und Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK2.

Der Vektor pCLiK2 wurde mit der Restriktionsendonuklease Dral (New England Biolabs, Beverly, USA) geschnitten. Nach Elektrophorese in einem 0,8%igen Agarosegel wurde ein ca. 2,3 kb großes Vektorfragment mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers religiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK3.

Ausgehend vom Plasmid pWLQ2 (Liebl et al., 1992) als Template für eine PCR Reaktion wurde mit den Oligonukleotidprimern SEQ ID NO:5 und SEQ ID NO:6 der Replikationsursprung pHM1519 amplifiziert.
SEQ ID NO:5:
   5'-GAGAGGGCGGCCGCGCAAAGTCCCGCTTCGTGAA-3'
SEQ ID NO:6:
   5'-GAGAGGGCGGCCGCTCAAGTCGGTCAAGCCACGC-3'

Neben den zu pWLQ2 komplementären Sequenzen, enthalten die Oligonukleotidprimer SEQ ID NO:5 und SEQ ID NO:6 Schnittstellen für die Restriktionsendonuklease Notl. Die PCR Reaktion wurde nach Standardmethode von Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 2,7 kb wurde mit dem GFX™PCR, DNA and Gel Band Purification Kit (Amersham Pharmacia, Freiburg) nach Angaben des Herstellers gereinigt. Das DNA-Fragment wurde mit der Restriktionsendonuklease Notl (New England Biolabs, Beverly, USA) geschnitten und im Anschluß daran erneut mit dem GFX™PCR, DNA and Gel Band Purification Kit (Amersham Pharmacia, Freiburg) nach Angaben des Herstellers gereinigt. Der Vektor pCLiK3 wurde ebenfalls mit der Restriktionsendonuklease Notl geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 2,3kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5.

Für die Erweiterung von pCLiK5 um eine "multiple cloning site" (MCS) wurden die beide synthetischen, weitestgehend komplementären Oligonukleotide SEQ ID NO:7 und SEQ ID NO:8, die Schnittstellen für die Restriktionsendonukleasen SwaI, XhoI, Aatl, Apal, Asp718, Mlul, Ndel, SpeI, EcoRV, SalI, ClaI, BamHI, Xbal und Smal enthalten, durch gemeinsames erhitzen auf 95°C und langsames abkühlen zu einem doppelsträngigen DNA-Fragment vereinigt.
SEQ ID NO:7:
SEQ ID NO:8:

Der Vektor pCLiK5 wurde mit den Restriktionsendonuklease Xhol und BamHI (New England Biolabs, Beverly, USA) geschnitten und mit alkalischer Phosphatase (I (Roche Diagnostics, Mannheim)) nach Angaben des Herstellers dephosphoryliert. Nach Elektrophorese in einem 0,8%igen Agarosegel wurde der linearisierte Vektor (ca. 5,0 kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem synthetischen Doppelsträngigen DNA-Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5MCS.

Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS ist als SEQ ID NO:9 aufgeführt.

### Beispiel 2

### Herstellung des Vektors pCLiK5MCS integrativ sacB

Ausgehend vom Plasmid pK19mob (Schäfer et al., Gene 145,69-73(1994)) als Template für eine PCR Reaktion wurde mit den Oligonukleotidprimem SEQ ID NO:10 und SEQ ID NO:11 das Bacillus subtilis sacB Gen amplifiziert.
SEQ ID NO:10:
   5'-GAGAGCGGCCGCCGATCCTTTTTAACCCATCAC-3'
SEQ ID NO:11:
   5'-AGGAGCGGCCGCCATCGGCATTTTCTTTTGCG-3`

Neben den zu pK19mobsac komplementären Sequenzen, enthalten die Oligonukleotidprimer SEQ ID NO:10 und SEQ ID NO:11 Schnittstellen für die Restriktionsendonuklease Notl. Die PCR Reaktion wurde nach Standardmethode von Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 1,9 kb wurde mit dem GFX™PCR, DNA and Gel Band Purification Kit (Amersham Pharmacia, Freiburg) nach Angaben des Herstellers gereinigt. Das DNA-Fragment wurde mit der Restriktionsendonuklease Notl (New England Biolabs, Beverly, USA) geschnitten und im Anschluß daran erneut mit dem GFX™PCR, DNA and Gel Band Purification Kit (Amersham Pharmacia, Freiburg) nach Angaben des Herstellers gereinigt. Der Vektor pCLiK5MCS wurde ebenfalls mit der Restriktionsendonuklease Notl geschnitten und mit alkalischer Phosphatase (I (Roche Diagnostics, Mannheim)) nach Angaben des Herstellers dephosphoryliert. Nach Elektrophorese in einem 0,8%igen Agarosegel wurde ein ungefähr 2,4 kb großes Vektorfragment mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5MCS integrativ sacB. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS integrativ sacB ist als SEQ ID NO:12 aufgeführt.

### Beispiel 3

### Isolierung und Klonierung des metK Gens aus C. glutamicum

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Die folgenden Oligonukleotidprimer wurden ausgehend von der metK Sequenz aus Großmann et al. (2000) FEMS Microbiology Letters 193:99-103 synthetisiert:
SEQ ID NO:13
   5'-GAGAGCCCGGGAAGAAGGGCTGCGACCTCCTCAT -3'
   und
SEQ ID NO:14
   5'-CTCTCACGCGTCATATGCAGGTGAGGTAACCCCA -3'

Nach Standardmethoden von Innis et al. (1990) PCR Protocols. A Guide to Methods and Applications, Academic Press, und unter Einsatz der aufgeführten Oligonukleotidprimer, sowie Pfu Turbo Polymerase (Fa. Stratagene) wurde ein DNA Fragment von 1640 Basenpaaren aus der genomischen DNA von C. glutamicum amplifiziert.

Das Fragment wurde mit den Restriktionsenzymen Mlu I und Sma I (Roche Diagnostics, Mannheim), die über die PCR-Oligonukleotidprimer eingebracht wurden, gespalten und gelelektrophoretisch aufgetrennt. Anschließend wurde das DNA Fragment mit GFX™PCR, DNA and Gel Band Purification Kit (Amersham Pharmacia, Freiburg) aus der Agarose aufgereinigt.

Der Vektor pCLiK5MCS, SEQ ID NO:9 wurde ebenfalls mit den Restriktionsenzymen Sma I und Mlu I gespalten und mit alkalischer Phosphatase I (Roche Diagnostics, Mannheim) nach Angaben des Herstellers dephosphoryliert. Vektor und DNA Fragment wurden mit T4 DNA Ligase (Amersham Pharmacia, Freiburg) ligiert und nach Standardmethoden wie in Sambrook et al. (1989), Molecular Cloning. A Laboratory Manual, Cold Spring Harbor, beschrieben, in E.coli XL-1 Blue (Fa. Stratagene) transformiert.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS/metKwt ist als SEQ ID NO:15 aufgeführt.

### Beispiel 4

### Mutagenese des metK Gens aus C. glutamicum

Die gerichtete Mutagenese des metK Gens aus C. glutamicum wurde mit dem QuickChange Kit (Fa. Stratagene) und nach Angaben des Herstellers durchgeführt. Die Mutagenese wurde im Plasmid pCLiK5MCS/metKwt, SEQ ID NO:15 durchgeführt. Für den Austausch von Cystein 94 aus SEQ ID NO: 16 nach Alanin 94 wurden folgende Oligonukleotidprimer synthetisiert:
SEQ ID NO:17
   5'- GATTCGACGGACGCACCGCTGGCGTCTCAGTATCCATC -3'
   und
SEQ ID NO:18
   5'-GATGGATACTGAGACGCCAGCGGTGCGTCCGTCGAATC -3'

Der Einsatz dieser Oligonukleotidprimer führt in SEQ ID NO:15 zu einem Austausch der Nukleotide in Position 1056 (von C nach G) und 1057 (von A nach C). Der resultierende Aminosäureaustausch Cys94Ala im metK Gen wurde nach Transformation und Plasmidpräparation durch Sequenzierungsreaktionen bestätigt. Das Plasmid erhielt die Bezeichnung pCLiK5MCS/metKC94A und ist als SEQ ID NO:19 aufgeführt.

### Beispiel 5

### SAM Synthetase (metK) Assay

C. glutamicum Stämme, die entweder mit dem Plasmid pCLiK5MCS/metKwt, SEQ ID NO: 15, oder mit dem Plasmid pCLiK5MCS/metKC94A, SEQ ID NO: 19 transformiert wurden, wurden in BHI/Glucose-Medium (37 g/l Brain Heart Infusion Fertigmedium, Fa. Difco, 10 mM (NH₄)₂SO₄, 4% Glucose) bei 30°C bis zu einer OD₆₀₀ von 20 angezogen. Die Zellen wurden bei 4°C abzentrifugiert und das Pellet wurde mit kalter physiologischer Kochsalzlösung gewaschen. Nach erneuter Zentrifugation wurden 0,25 g feuchtes Zellpellet mit 1 mL Aufschlußpuffer (50 mM Tris pH 7,5, 10 mM MgCl₂, 50 mM KCl, 1 mM DTT) bei 4°C resuspendiert. In einem Ribolyser der Fa. Hybaid und in blauen Ribolyserröhrchen der Fa. Hybaid und bei einer Rotationseinstellung von 6,0 wurde die Bakteriensuspension dreimal für jeweils 30 Sekunden lysiert. Das Lysat wurde durch 45minütige Zentrifugation bei 13.000 rpm in einer Eppendorfzentrifuge geklärt und der Überstand 1:10 mit Wasser verdünnt. Der Proteingehalt wurde nach Bradford, M. M. (1976) Anal. Biochem. 72:248-254 bestimmt.

Die enzymatische Aktivität der SAM Synthase wurde nach Markham, G.D. et al. (1983) Methods in Enzymology 94: 219-222, mit folgenden Modifikationen bestimmt.

Reaktionsansätze von 100 µl mit 100 mM Tris pH 8.0, 100 mM KCI, 20 mM MgCl₂, 1,2 mM L-Methionin, 10 mM ATP, 1 µl ³⁵S-L-Methionin, entsprechend 15,15 µCi (Amersham SJ204, spez. Aktivität 1 Ci/µmol) und H₂O ad 100 µl wurden mit 100 µg der jeweiligen Proteinlysate gestartet und bei 37°C inkubiert. Zu den Zeitpunkten 0, 5, 10, 20, 30 und 60 Minuten wurden 10 µl Aliquots des Reaktionsansatzes entnommen und auf Eis mit 20 µl 50 mM EDTA gestoppt.

30 µl der abgestoppten Reaktion wurden auf Phosphocellulose Filtereinheiten (Fa. Pierce, Nr. 29520) gegeben und bei 6.000 rpm in einer Eppendorf-Zentrifuge für 1 Minute abzentrifugiert. Der Filter wurde zweimal mit 500 µl 75 mM Phosphorsäure gewaschen und dann in ein Zählröhrchen mit Szintillationsflüssigkeit gegeben. Die Radioaktivität des gebildeten S-Adenosylmethionins wird in einem Szintilationszähler (Fa. Beckman) bestimmt.

Die Messergebnisse sind in beiliegender Figur 1 dargestellt.

Unter Berücksichtigung der spezifischen Aktivität des radioaktiven L-Methioninssowie der eingesetzten Proteinmenge läßt sich die Rate der S-Adenosylmethionin-Bildung aus der Zunahme der eingebauten Radioaktivität pro Zeiteinheit bestimmen. Ihre Einheit lautet µmol S-Adenosylmethionin/min*mg Protein. Diese Rate kann zwischen Wildtypenzym und Mutantenenzym verglichen werden.

### Beispiel 6

### Bestimmung des zellulären S-Adenosylmethionin Titers in C. glutamicum

Zur Bestimmung der zellulären Titer von S-Adenosylmethionin in C. glutamicum Stämmen, die entweder mit pCLiK5MCS/metKwt (SEQ ID NO:15), oder pCLiK5MCS/metKC94A (SEQ ID NO:19) transformiert wurden, wurde wie folgt verfahren. Ein wie in Beispiel 5 erhaltenes Zellpellet, das mit eiskalter physiologischer Kochsalzlösung gewaschen wurde, wurde mit Trichloressigsäure (200 µl TCA pro 0,1 g feuchtes Pellet) resuspendiert. Nach 5 Minuten auf Eis wurde die Suspension bei 4°C und 13.000 rpm für 5 min in einer Eppendorfzentrifuge geklärt. Der S-Adenosylmethionin Gehalt im Überstand wurde mittels HPLC bestimmt (Ionospher 5C Kationenaustauschersäule, 10 µl Injektionsvolumen; Laufmittel: 70% vol/vol 0,2 M Ammoniumformiat pH 4,0 30% vol/vol Methanol; UV-Detektion 260 nm; 40°C; Retentionszeit 8,5 Min.).

**Tab.1. S-Adenosylmethionin-Titer**

| | mg/l |
|---|---|
| ATCC 13032 + metK | 73,94 |
| ATCC 13032 + metK C94A | 47,36 |

### Beispiel 7

### Austausch des metK wt Gens in C. glutamicum durch metK C94A

Für den allelischen Austausch des metK Wildtypgens in C. glutamicum KFCC10065 durch die Mutante metK C94A wurde zunächst die metK C94A Sequenz aus SEQ ID NO:19 in pCLiKSMCS integrativ sacB (SEQ ID NO:12) kloniert. Dazu wurde das Plasmid pCLiK5MCS/metKC94A (SEQ ID NO:19) mit den Restriktionsendonukleasen Bgl II und Xho I (Fa. NEB, Schwalbach) gespalten. Das erhaltene Fragment der Größe 1962 Basenpaare wurde wie in Beispiel 3 beschrieben, gereinigt. Der Vektor pCLiK5MCS integrativ sacB wurde ebenfalls mit Bgl II und Xhol gespalten und wie in Beispiel 3 beschrieben, aufgereinigt. Vektor und Fragment wurden wie in Beispiel 3 beschrieben, ligiert, in E.coli XL-1Blue transformiert. Das Plasmid wurden gereinigt und nach Sequenzierung bestätigt. Das erhaltene Plasmid pCLiK5MCS integrativ sacB/metKC94A ist als SEQ ID NO:20 aufgeführt.

Das Plasmid pCLiK5MCS integrativ sacB/metKC94A wurde in C. glutamicum KFCC10065 mittels Elektroporation wie bei Liebl, et al. (1989) FEMS Microbiology Letters 53:299-303 beschrieben, transformiert. Modifikationen des Protokolls sind in DE 10046870 beschrieben. Die chromosomale Anordnung des metK-Lokus einzelner Transformanten wurde mit Standardmethoden durch Southernblot und Hybridisierung wie in Sambrook et al. (1989), Molecular Cloning. A Laboratory Manual, Cold Spring Harbor, beschrieben, überprüft. Dadurch wurde sichergestellt, daß es sich bei den Transformanten um solche handelt, die das transformierte Plasmid durch homologe Rekombination am metK-Lokus integriert haben. Nach Wachstum solcher Kolonien über Nacht in Medien, die kein Antibiotikum enthalten, werden solche Transformanten dann auf ein Saccharose-CM-Agarmedium (10% Saccharose) ausplattiert und bei 30°C für 24 Stunden inkubiert.

Da das im Vektor pCLiK5MCS integrativ sacB/metKC94A enthaltende sacB Gen Saccharose in ein toxisches Produkt umwandelt, können nur solche Kolonien anwachsen, die das sacB Gen durch einen zweiten homologen Rekombinationsschritt zwischen dem Wildtyp metK Gen und dem mutierten metKC94A Gen deletiert haben. Während der homologen Rekombination kann entweder das Wildtyp Gen, oder das mutierte Gen zusammen mit dem sacB Gen deletiert werden. Wenn das sacB Gen zusammen mit dem Wildtyp Gen entfernt wird, resultiert eine mutierte Transformante.

Anwachsende Kolonien wurden gepickt, ihre genomische DNA präpariert und das metK Gen mit zwei Methoden analysiert. Zum einen wurde der Austausch von zwei Nukleotiden, wie in Beispiel 4 beschrieben, genutzt. Mit Hilfe eines spezifischen PCR-Oligonukleotidprimers, der an seinem 3'-Ende zwischen beiden Allelen differenzieren kann und eines zweiten metKspezifischen Oligonukleotidprimers konnten diagnostische PCR-Fragmente generiert werden. Zum anderen wurden bei etwa 100 Transformanten der metK-Lokus nach PCR-Amplifikation mit PCR-Oligonukleotidprimern, die stromauf, bzw. stromab der Mutation binden, sequenziert. Es wurden mehrere mutierte metK Klone erhalten. Ein solcher Klon wurde mit KFCC10065metKC94A bezeichnet. Die Aminosäuresequenz der Mutante C94A entspricht SEQ ID NO:22.

### Beispiel 8

### Herstellung von Methionin mit dem Stamm KFCC10065metKC94A

Der in Beispiel 6 hergestellte Stamm KFCC10065metKC94A wurde auf einer Agar Platte mit BHI-Medium (Difco) für 2 Tage bei 30°C angezogen. Die aufgewachsenen Zellen wurden in Saline von der Agarplatte suspendiert und mit einer OD 600nm von 1,5 in das Medium II überführt. Das Medium II wurde wie folgt zusammengesetzt.

### Medium IIA

| | |
|---|---|
| 0.6g/l | KH₂PO₄ |
| 0.4g/l | MgSO₄*7H₂O |
| 25g/l | (NH₄)₂SO₄ |
| 40g/l | Rohzucker |
| 60g/l | Melasse |

Das so angesetzte Medium wurde mit NH₄OH auf pH 7,8 eingestellt und bei 120°C für 30 min sterilisiert.

### Medium IIB:

| | |
|---|---|
| 0.3mg/l | Thiamin*HCl |
| 1mg/l | Biotin |
| 2mg/l | FeSO₄ |
| 2mg/l | MnSO₄ |
| 0.1mg/l | Vit.B12 |

Medium IIB wurden separat angesetzt, durch Filtration sterilisiert und dem Medium IIA zugefügt. Beide Bestandteile IIa und IIB ergeben zusammen das Medium II.

Vom Medium II (= IIA+B) wurden 10ml in einem 100ml Erlenmyerkolben mit 0,5g sterilisiertem CaCO₃ mit Zellen des oben genannten Stamms versetzt, für 72h auf einem Orbitalschüttler mit 200 Upm bei 30°C inkubiert.

Gebildetes Methionin in der Kulturbrühe wurde mit Hilfe der Aminosäuresäure-Bestimmungsmethode von Agilent auf einer Agilent 1100 Series LC System HPLC bestimmt. Eine Vorsäulenderivatisierung mit Ortho-Phthalaldehyd erlaubt die Quantifizierung der gebildeten Aminosäuren, die Auftrennung des Aminosäuregemsich findet auf einer Hypersil AA-Säule (Agilent) statt.

## Patentansprüche

1. Verfahren zur fermentativen Herstellung von L-Methionin, welches folgende Schritte umfasst:
a) Fermentation einer L-Methionin produzierenden coryneformen Bakterienkultur, wobei in den coryneformen Bakterien zumindest eine Nukleotidsequenz exprimiert wird, welche für ein Protein mit gegenüber dem Wildtyp-Enzym verringerter S-Adenosylmethionin Synthase (metK)-Aktivität kodiert, wobei die metk-kodierende Sequenz eine Nukleotidsequenz ist, welche für ein Protein mit verringerter metK-Aktivität kodiert, in welchem wenigstens ein Cysteinrest des Wildtypproteins substituiert ist,
b) Anreicherung der schwefelhaltigen Feinchemikalie im Medium und/oder in den Zellen der Bakterien, und
c) Isolieren der schwefelhaltigen Feinchemikalie
wobei die metK-kodierende Sequenz eine kodierende Nukleotidsequenz ist, die für ein Protein mit metK-Aktivität kodiert, welches folgende Aminosäureteilsequenz aufweist:
G(F/Y) (D/S)X¹X²(S/T)X³(G/A)V
worin
X¹ und X² unabhängig voneinander für eine beliebige Aminosäure stehen;
und
X³ für eine von Cys verschiedene durch Mutation eingeführte Aminosäure steht.

2. Verfahren Anspruch 1, wobei die metK-kodierende Sequenz für ein Protein mit metK-Aktivität kodiert, wobei das Protein eine Aminosäuresequenz von Met1 bis Ala407 gemäß SEQ ID NO: 22 umfasst.

3. Verfahren nach einem der vorhergehenden Ansprüche, wobei die kodierende metK-Sequenz eine in coryneformen Bakterien replizierbare oder eine stabil in das Chromosom integrierte DNA oder eine RNA ist.

4. Verfahren gemäß einem der vorhergehenden Ansprüche, wobei man
a) einen mit einem Plasmidvektor transformierten Bakterienstamm einsetzt, der wenigstens eine Kopie der mutierten metK-Sequenz unter Kontrolle regulativer Sequenzen trägt, oder
b) einen Stamm einsetzt, in dem die mutierte metK-Sequenz in das in das Chromosom des Bakteriums integriert wurde.

5. Verfahren gemäß einem der vorhergehenden Ansprüche, wobei man coryneforme Bakterien fermentiert, in denen gleichzeitig wenigstens eines der Gene, ausgewählt unter
a) dem für eine Aspartatkinase kodierenden Gen lysC,
b) dem für eine Aspartat-Semialdehyd-Dehdrogenase kodierende Gen asd
c) dem für die Glycerinaldehyd-3-Phosphat Dehydrogenase kodierende Gen gap,
d) dem für die 3-Phosphoglycerat Kinase kodierende Gen pgk,
e) dem für die Pyruvat Carboxylase kodierende Gen pyc,
f) dem für die Triosephosphat Isomerase kodierende Gen tpi,
g) dem für die Homoserin O-Acetyltransferase kodierende Gen metA,
h) dem für die Dystahionin-gamma-Synthase kodierende Gen metB
i) dem für die Cystahionin-gamma-Lyase kodierende Gen metC,
j) dem für die Methionin-Synthase kodierenden Gen metH
k) dem für die Serin-Hydroxymethyltransferase kodierenden Gen glyA,
l) dem für die O-Acetylhomoserin-Sulfhydrylase kodierende Gen metY,
m) dem für die Metylentetrahydrofolat-Reduktase kodierenden Gens metF Gen,
n) dem für die Phosphoserin-Aminotransferase kodierenden Gens serC Gen, das für die kodiert,
o) dem für die Phosphoserin-Phosphatase kodierenden Gens serB,
p) dem für die Serin Acetyl-Transferase kodierenden Gens cysE,
q) dem für die Cystein-Synthase kodierenden Gen cysK, und
r) dem für die Homoserin-Dehydrogenase kodierenden Gens hom überexprimiert ist; und/oder
in denen gleichzeitig wenigstens eines dieser Gene so mutiert ist, dass das korrespondierende Protein, verglichen mit dem nicht mutierten Protein in geringerem Maße oder nicht mehr durch einen Stoffwechselmetaboliten in seiner Aktivität beeinflusst wird, oder dass dessen spezifischen Aktivität gesteigert wird.

6. Verfahren gemäß einem der vorhergehenden Ansprüche, wobei man coryneforme Bakterien fermentiert, in denen gleichzeitig wenigstens eines der Gene, ausgewählt unter
a) dem für die Homoserine-Kinase kodierenden Gen thrB,
b) dem für Threonin Dehydrogenase kodierenden Gen ilvA,
c) dem für die Threonin Synthase kodierenden Gen thrC
d) dem für die Meso-Diaminopimelat D-Dehydrogenase kodierenden Gen ddh
e) dem für die Phosphoenopyruvat-Carboxykinase kodierenden Gen pck,
f) dem für die Glucose-6-Phosphat-6-Isomerase kodierenden Gen pgi,
g) dem für die Pyruvat-Oxidase kodierenden Gen poxB,
h) dem für die Dihydrodipicolinat Synthase kodierenden Gen dapA,
i) dem für die Dihydrodipicolinat Reduktase kodierenden Gen dapB; oder
j) dem für die Diaminopicolinat Decarboxylase kodierenden Gen lysA
abschwächt ist; und/oder
wobei man coryneforme Bakterien fermentiert, in denen gleichzeitig wenigstens eines dieser Gene so mutiert ist, so dass die enzymatische Aktivität des korrespondierenden Proteins teilweise oder vollständig verringert wird.

7. Verfahren gemäß einem oder mehreren der vorhergehenden Ansprüche, wobei man Mikroorganismen der Art Corynebacterium glutamicum einsetzt.

8. Verfahren zur Herstellung eines L-Methionin haltigen Tierfuttermittel-Additivs aus Fermentationsbrühen, welches folgende Schritte umfasst
a) Kultivierung und Fermentation eines L-Methionin produzierenden Mikroorganismus gemäß der Definition in einem der vorhergehenden Ansprüche in einem Fermentationsmedium;
b) Entfernung von Wasser aus der L-Methionin haltigen Fermentationsbrühe;
c) Entfernung der während der Fermentation gebildeten Biomasse in einer Menge von 0 bis 100 Gew.-%; und
d) Trocknung der gemäß b) und/oder c) erhaltenen Fermentationsbrühe, um das Tierfuttermittel-Additiv in der gewünschten Pulver- oder Granulatform zu erhalten.

## Claims

1. Method for the L-methionine by fermentation production of L-methionine, which comprises the following steps:
a) fermentation of a coryneform bacterial culture which produces, the coryneform bacteria expressing at least one nucleotide sequence which encodes a protein with, S-adenosylmethionine synthase (metK) activity which is reduced in comparison with the wild type enzyme wherein the metK-encoding sequence is a nucleotide sequence which encodes a protein with reduced metK activity in which at least one cysteine residue of the wild-type protein is substituted,
b) enrichment of the sulfur-containing fine chemical in the medium and/or in the bacterial cells, and
c) isolation of the sulfur-containing fine chemical,
wherein the metK-encoding sequence is a coding nucleotide sequence which encodes a protein with metK activity which has the following amino acid part-sequence:
G(F/Y)(D/S)X¹X²(S/T)X³(G/A)V
in which
X¹ and X² independently of one another represent any amino acid;
and
X³ represents an amino acid other than Cys introduced by mutation.

2. Method according to Claim 1, wherein the metK-encoding sequence encodes a protein with metK activity, the protein encompassing an amino acid sequence from Met1 to Ala407 as shown in SEQ ID NO: 22.

3. Method according to any of the preceding claims, wherein the coding metK sequence is a DNA which is capable of replication in coryneform bacteria or stably integrated into the chromosome, or an RNA.

4. Method according to any of the preceding claims, wherein
a) a bacterial strain which has been transformed with a plasmid vector and which carries at least one copy of the mutated metK sequence under the control of regulatory sequences is used, or
b) a strain in which the mutated metK sequence has been integrated into the bacterial chromosome is used.

5. Method according to any of the preceding claims, wherein coryneform bacteria in which at least one of the genes selected from among
a) the gene lysC, which encodes an aspartate kinase,
b) the gene asd, which encodes an aspartate-semialdehyde dehydrogenase,
c) the gene gap, which encodes glycerinaldehyde-3-phosphate dehydrogenase,
d) the gene pgk, which encodes 3-phosphoglycerate kinase,
e) the gene pyc, which encodes pyruvate carboxylase,
f) the gene tpi, which encodes triose-phosphate isomerase,
g) the gene metA, which encodes homoserine 0-acetyltransferase,
h) the gene metB, which encodes cystathionine-gamma synthase,
i) the gene metC, which encodes cystathionine-gamma lyase,
j) the gene metH, which encodes methionine synthase,
k) the gene glyA, which encodes serine hydroxymethyltransferase,
l) the gene metY, which encodes 0-acetylhomoserine sulfhydrylase,
m) the gene metF, which encodes methylenetetrahydrofolate reductase,
n) the gene serC, which encodes phosphoserine aminotransferase,
o) the gene serB, which encodes phosphoserine phosphatase,
p) the gene cysE, which encodes serine acetyltransferase,
q) the gene cysK, which encodes cysteine synthase,
r) the gene hom, which encodes homoserine dehydrogenase,
is simultaneously overexpressed are fermented; and/or
in which at least one of these genes is simultaneously mutated in such a way that the activity of the corresponding protein, in comparison with the unmutated protein, is influenced to a lesser degree or no longer by a metabolite, or in such a way that its specific activity is increased.

6. Method according to any of the preceding claims, wherein coryneform bacteria are fermented in which simultaneously at least one of the genes selected from among
a) the gene thrB, which encodes homoserine kinase,
b) the gene ilvA, which encodes threonine dehydrogenase,
c) the gene thrC, which encodes threonine synthase,
d) the gene ddh, which encodes meso-diaminopimelate D dehydrogenase,
e) the gene pck, which encodes phosphoenol-pyruvate carboxykinase,
f) the gene pgi, which encodes glucose-6-phosphate-6 isomerase,
g) the gene poxB, which encodes pyruvate oxidase,
h) the gene dapA, which encodes dihydrodipicolinate synthase,
i) the gene dapB, which encodes dihydrodipicolinate reductase, or
j) the gene lysA, which encodes diaminopicolinate decarboxylase,
is attenuated; and/or
wherein coryneform bacteria are fermented in which at least one of these genes is simultaneously mutated in such a way that the enzyme activity of the corresponding protein is reduced in part or fully.

7. Method according to one or more of the preceding claims, wherein microorganisms of the species Corynebacterium glutamicum are used.

8. Method for producing an L-methionine-containing animal feed additive from fermentation broths, which comprises the following steps:
a) culturing and fermentation of an L-methionine-producing microorganism as defined in any of the preceding claims in a fermentation medium;
b) removal of water from the L-methionine-containing fermentation broth;
c) removal of from 0 to 100% by weight of the biomass formed during fermentation; and
d) drying of the fermentation broth obtained according to b) and/or c), in order to obtain the animal feed additive in the desired powder or granule form.

## Revendications

1. Procédé de fabrication par fermentation de L-méthionine, qui comprend les étapes suivantes :
a) fermentation d'une culture de bactéries corynéformes produisant la L-méthionine, au moins une séquence nucléotidique qui code pour une protéine ayant une activité de S-adénosylméthionine-synthase (metK) diminuée par rapport à celle de l'enzyme de type sauvage, étant exprimée dans les bactéries corynéformes, la séquence codant pour metK étant une séquence nucléotidique qui code pour une protéine ayant une activité de metK diminuée, dans laquelle au moins un résidu de cystéine de la protéine de type sauvage est substitué,
b) enrichissement du produit de la chimie fine, contenant du soufre, dans le milieu et/ou dans les cellules des bactéries, et
c) isolement du produit de la chimie fine contenant du soufre,
la séquence codant pour metK étant une séquence nucléotidique codante qui code pour une protéine ayant une activité de metK, qui a la séquence partielle d'acides aminés suivante :
G(F/Y) (D/S)X¹X²(S/T)X³(G/A)V
dans laquelle
X¹ et X² représentent chacun indépendamment de l'autre un acide aminé quelconque,
et
X³ est un acide aminé, différent de Cys, introduit par mutation.

2. Procédé selon la revendication 1, dans lequel la séquence codant pour metK code pour une protéine ayant une activité de metK, la protéine comprenant une séquence d'acides aminés de Met1 à Ala407 selon SEQ ID N° 22.

3. Procédé selon l'une des revendications précédentes, dans lequel la séquence metK codante est un ADN réplicable dans les bactéries corynéformes ou intégré d'une manière stable dans le chromosome, ou un ARN.

4. Procédé selon l'une des revendications précédentes, dans lequel
a) on utilise une souche bactérienne, transformée avec un vecteur plasmidique, et qui porte au moins une copie de la séquence metK mutée sous le contrôle de séquences régulatrices, ou
b) on utilise une souche dans laquelle la séquence metK mutée a été intégrée dans le chromosome de la bactérie.

5. Procédé selon l'une des revendications précédentes, dans lequel on fermente des bactéries corynéformes dans lesquelles simultanément au moins l'un des gènes choisis parmi :
a) le gène lysC, codant pour une aspartatekinase,
b) le gène asd, codant pour une aspartate-semi-aldéhyde-déshydrogénase,
c) le gène gap, codant pour la glycérinaldéhyde-3-phosphate-déshydrogénase,
d) le gène pgk, codant pour la 3-phosphoglycérate-kinase,
e) le gène pyc, codant pour la pyruvate-carboxylase,
f) le gène tpi, codant pour la triosephosphate-isomérase,
g) le gène metA, codant pour l'homosérine-0-acétyltransférase,
h) le gène metB, codant pour la cystathionine-gamma-synthase,
i) le gène metC, codant pour la cystathionine-gamma-lyase,
j) le gène metH, codant pour la méthionine-synthase,
k) le gène glyA, codant pour la sérine-hydroxyméthyltransférase,
l) le gène metY, codant pour l'O-acétylhomosérine-sulfhydrylase,
m) le gène metF, codant pour la méthylène-tétrahydrofolate-réductase,
n) le gène serC, codant pour la phosphosérine-aminotransférase,
o) le gène serB, codant pour la phosphosérine-phosphatase,
p) le gène cysE, codant pour la sérine-acétyl-transférase,
q) le gène cysK, codant pour la cystéine-synthase, et
r) le gène hom, codant pour l'homosérine-déshydrogénase
est surexprimé; et/ou
dans lesquelles l'un de ces gènes est muté de telle sorte que son activité soit influencée, ou que son activité spécifique soit augmentée, d'une manière plus faible que dans le cas de la protéine non mutée, ou ne le soit plus sous l'effet d'un métabolite.

6. Procédé selon l'une des revendications précédentes, dans lequel on fermente des bactéries corynéformes dans lesquelles simultanément au moins l'un des gènes choisis parmi :
a) le gène thrB, codant pour l'homosérine-kinase,
b) le gène ilvA, codant pour la thréonine-déshydrogénase,
c) le gène thrC, codant pour la thréonine-synthase,
d) le gène ddh, codant pour la méso-diaminopimélate-D-déshydrogénase,
e) le gène pck, codant pour la phosphoénopyruvate-carboxykinase,
f) le gène pgi, codant pour la glucose-6-phosphate-6-isomérase,
g) le gène poxB, codant pour la pyruvate-oxydase,
h) le gène dapA, codant pour la dihydrodipicolinate-synthase,
i) le gène dapB, codant pour la dihydrodipicolinate-réductase; ou
j) le gène lysA, codant pour la diaminopicolinate-décarboxylase ;
est atténué ; et/ou
dans lesquelles on fermente des bactéries corynéformes dans lesquelles simultanément l'un de ces gènes est muté de telle sorte que l'activité enzymatique de l'enzyme correspondante soit partiellement ou complètement diminuée.

7. Procédé selon l'une ou plusieurs des revendications précédentes, dans lequel on utilise des micro-organismes de l'espèce Corynebacterium glutamicum.

8. Procédé de fabrication d'un additif pour aliments pour animaux, contenant de la L-méthionine, à partir de bouillons de fermentation, qui comprend les étapes suivantes :
a) culture et fermentation d'un micro-organisme produisant de la L-méthionine selon la définition donnée dans l'une des revendications précédentes, dans un milieu de fermentation ;
b) élimination de l'eau se trouvant dans le bouillon de fermentation contenant de la L-méthionine ;
c) élimination, en une quantité de 0 à 100 % en poids, de la biomasse formée au cours de la fermentation ; et
d) séchage du bouillon de fermentation obtenu selon b) et/ou c), pour obtenir l'additif pour aliments pour animaux, sous la forme pulvérulente ou granulée souhaitée.


### SEQUENCE LISTING

<110> BASF Aktiengesellschaft
<120> METK
<130> M/43138
<160> 23
<170> PatentIn version 3.1
<210> 1
   <211> 52
   <212> DNA
   <213> Künstliche Sequenz
<400> 1
   cccgggatcc gctagcggcg cgccggccgg cccggtgtga aataccgcac ag 52
<210> 2
   <211> 53
   <212> DNA
   <213> Künstliche Sequenz
<400> 2
   tctagactcg agcggccgcg gccggccttt aaattgaaga cgaaagggcc tcg 53
<210> 3
   <211> 47
   <212> DNA
   <213> Künstliche Sequenz
<400> 3
   gagatctaga cccggggatc cgctagcggg ctgctaaagg aagcgga 47
<210> 4
   <211> 38
   <212> DNA
   <213> Künstliche Sequenz
<400> 4
   gagaggcgcg ccgctagcgt gggcgaagaa ctccagca 38
<210> 5
   <211> 34
   <212> DNA
   <213> Künstliche Sequenz
<400> 5
   gagagggcgg ccgcgcaaag tcccgcttcg tgaa 34
<210> 6
   <211> 34
   <212> DNA
   <213> Künstliche Sequenz
<400> 6
   gagagggcgg ccgctcaagt cggtcaagcc acgc 34
<210> 7
   <211> 140
   <212> DNA
   <213> Künstliche Sequenz
<400> 7
   tcgaatttaa atctcgagag gcctgacgtc gggcccggta ccacgcgtca tatgactagt 60
   tcggacctag ggatatcgtc gacatcgatg ctcttctgcg ttaattaaca attgggatcc 120
   tctagacccg ggatttaaat 140
<210> 8
   <211> 140
   <212> DNA
   <213> Künstliche Sequenz
<400> 8
   gatcatttaa atcccgggtc tagaggatcc caattgttaa ttaacgcaga agagcatcga 60
   tgtcgacgat atccctaggt ccgaactagt catatgacgc gtggtaccgg gcccgacgtc 120
   aggcctctcg agatttaaat 140
<210> 9
   <211> 5091
   <212> DNA
   <213> Künstliche Sequenz
<400> 9
   tcgatttaaa tctcgagagg cctgacgtcg ggcccggtac cacgcgtcat atgactagtt 60
   cggacctagg gatatcgtcg acatcgatgc tcttctgcgt taattaacaa ttgggatcct 120
   ctagacccgg gatttaaatc gctagcgggc tgctaaagga agcggaacac gtagaaagcc 180
   agtccgcaga aacggtgctg accccggatg aatgtcagct actgggctat ctggacaagg 240
   gaaaacgcaa gcgcaaagag aaagcaggta gcttgcagtg ggcttacatg gcgatagcta 300
   gactgggcgg ttttatggac agcaagcgaa ccggaattgc cagctggggc gccctctggt 360
   aaggttggga agccctgcaa agtaaactgg atggctttct tgccgccaag gatctgatgg 420
   cgcaggggat caagatctga tcaagagaca ggatgaggat cgtttcgcat gattgaacaa 480
   gatggattgc acgcaggttc tccggccgct tgggtggaga ggctattcgg ctatgactgg 540
   gcacaacaga caatcggctg ctctgatgcc gccgtgttcc ggctgtcagc gcaggggcgc 600
   ccggttcttt ttgtcaagac cgacctgtcc ggtgccctga atgaactgca ggacgaggca 660
   gcgcggctat cgtggctggc cacgacgggc gttccttgcg cagctgtgct cgacgttgtc 720
   actgaagcgg gaagggactg gctgctattg ggcgaagtgc cggggcagga tctcctgtca 780
   tctcaccttg ctcctgccga gaaagtatcc atcatggctg atgcaatgcg gcggctgcat 840
   acgcttgatc cggctacctg cccattcgac caccaagcga aacatcgcat cgagcgagca 900
   cgtactcgga tggaagccgg tcttgtcgat caggatgatc tggacgaaga gcatcagggg 960
   ctcgcgccag ccgaactgtt cgccaggctc aaggcgcgca tgcccgacgg cgaggatctc 1020
   gtcgtgaccc atggcgatgc ctgcttgccg aatatcatgg tggaaaatgg ccgcttttct 1080
   ggattcatcg actgtggccg gctgggtgtg gcggaccgct atcaggacat agcgttggct 1140
   acccgtgata ttgctgaaga gcttggcggc gaatgggctg accgcttcct cgtgctttac 1200
   ggtatcgccg ctcccgattc gcagcgcatc gccttctatc gccttcttga cgagttcttc 1260
   tgagcgggac tctggggttc gaaatgaccg accaagcgac gcccaacctg ccatcacgag 1320
   atttcgattc caccgccgcc ttctatgaaa ggttgggctt cggaatcgtt ttccgggacg 1380
   ccggctggat gatcctccag cgcggggatc tcatgctgga gttcttcgcc cacgctagcg 1440
   gcgcgccggc cggcccggtg tgaaataccg cacagatgcg taaggagaaa ataccgcatc 1500
   aggcgctctt ccgcttcctc gctcactgac tcgctgcgct cggtcgttcg gctgcggcga 1560
   gcggtatcag ctcactcaaa ggcggtaata cggttatcca cagaatcagg ggataacgca 1620
   ggaaagaaca tgtgagcaaa aggccagcaa aaggccagga accgtaaaaa ggccgcgttg 1680
   ctggcgtttt tccataggct ccgcccccct gacgagcatc acaaaaatcg acgctcaagt 1740
   cagaggtggc gaaacccgac aggactataa agataccagg cgtttccccc tggaagctcc 1800
   ctcgtgcgct ctcctgttcc gaccctgccg cttaccggat acctgtccgc ctttctccct 1860
   tcgggaagcg tggcgctttc tcatagctca cgctgtaggt atctcagttc ggtgtaggtc 1920
   gttcgctcca agctgggctg tgtgcacgaa ccccccgttc agcccgaccg ctgcgcctta 1980
   tccggtaact atcgtcttga gtccaacccg gtaagacacg acttatcgcc actggcagca 2040
   gccactggta acaggattag cagagcgagg tatgtaggcg gtgctacaga gttcttgaag 2100
   tggtggccta actacggcta cactagaagg acagtatttg gtatctgcgc tctgctgaag 2160
   ccagttacct tcggaaaaag agttggtagc tcttgatccg gcaaacaaac caccgctggt 2220
   agcggtggtt tttttgtttg caagcagcag attacgcgca gaaaaaaagg atctcaagaa 2280
   gatcctttga tcttttctac ggggtctgac gctcagtgga acgaaaactc acgttaaggg 2340
   attttggtca tgagattatc aaaaaggatc ttcacctaga tccttttaaa ggccggccgc 2400
   ggccgcgcaa agtcccgctt cgtgaaaatt ttcgtgccgc gtgattttcc gccaaaaact 2460
   ttaacgaacg ttcgttataa tggtgtcatg accttcacga cgaagtacta aaattggccc 2520
   gaatcatcag ctatggatct ctctgatgtc gcgctggagt ccgacgcgct cgatgctgcc 2580
   gtcgatttaa aaacggtgat cggatttttc cgagctctcg atacgacgga cgcgccagca 2640
   tcacgagact gggccagtgc cgcgagcgac ctagaaactc tcgtggcgga tcttgaggag 2700
   ctggctgacg agctgcgtgc tcggccagcg ccaggaggac gcacagtagt ggaggatgca 2760
   atcagttgcg cctactgcgg tggcctgatt cctccccggc ctgacccgcg aggacggcgc 2820
   gcaaaatatt gctcagatgc gtgtcgtgcc gcagccagcc gcgagcgcgc caacaaacgc 2880
   cacgccgagg agctggaggc ggctaggtcg caaatggcgc tggaagtgcg tcccccgagc 2940
   gaaattttgg ccatggtcgt cacagagctg gaagcggcag cgagaattat cgcgatcgtg 3000
   gcggtgcccg caggcatgac aaacatcgta aatgccgcgt ttcgtgtgcc gtggccgccc 3060
   aggacgtgtc agcgccgcca ccacctgcac cgaatcggca gcagcgtcgc gcgtcgaaaa 3120
   agcgcacagg cggcaagaag cgataagctg cacgaatacc tgaaaaatgt tgaacgcccc 3180
   gtgagcggta actcacaggg cgtcggctaa cccccagtcc aaacctggga gaaagcgctc 3240
   aaaaatgact ctagcggatt cacgagacat tgacacaccg gcctggaaat tttccgctga 3300
   tctgttcgac acccatcccg agctcgcgct gcgatcacgt ggctggacga gcgaagaccg 3360
   ccgcgaattc ctcgctcacc tgggcagaga aaatttccag ggcagcaaga cccgcgactt 3420
   cgccagcgct tggatcaaag acccggacac ggagaaacac agccgaagtt ataccgagtt 3480
   ggttcaaaat cgcttgcccg gtgccagtat gttgctctga cgcacgcgca gcacgcagcc 3540
   gtgcttgtcc tggacattga tgtgccgagc caccaggccg gcgggaaaat cgagcacgta 3600
   aaccccgagg tctacgcgat tttggagcgc tgggcacgcc tggaaaaagc gccagcttgg 3660
   atcggcgtga atccactgag cgggaaatgc cagctcatct ggctcattga tccggtgtat 3720
   gccgcagcag gcatgagcag cccgaatatg cgcctgctgg ctgcaacgac cgaggaaatg 3780
   acccgcgttt tcggcgctga ccaggctttt tcacataggc tgagccgtgg ccactgcact 3840
   ctccgacgat cccagccgta ccgctggcat gcccagcaca atcgcgtgga tcgcctagct 3900
   gatcttatgg aggttgctcg catgatctca ggcacagaaa aacctaaaaa acgctatgag 3960
   caggagtttt ctagcggacg ggcacgtatc gaagcggcaa gaaaagccac tgcggaagca 4020
   aaagcacttg ccacgcttga agcaagcctg ccgagcgccg ctgaagcgtc tggagagctg 4080
   atcgacggcg tccgtgtcct ctggactgct ccagggcgtg ccgcccgtga tgagacggct 4140
   tttcgccacg ctttgactgt gggataccag ttaaaagcgg ctggtgagcg cctaaaagac 4200
   accaagggtc atcgagccta cgagcgtgcc tacaccgtcg ctcaggcggt cggaggaggc 4260
   cgtgagcctg atctgccgcc ggactgtgac cgccagacgg attggccgcg acgtgtgcgc    4320
   ggctacgtcg ctaaaggcca gccagtcgtc cctgctcgtc agacagagac gcagagccag    4380
   ccgaggcgaa aagctctggc cactatggga agacgtggcg gtaaaaaggc cgcagaacgc    4440
   tggaaagacc caaacagtga gtacgcccga gcacagcgag aaaaactagc taagtccagt    4500
   caacgacaag ctaggaaagc taaaggaaat cgcttgacca ttgcaggttg gtttatgact    4560
   gttgagggag agactggctc gtggccgaca atcaatgaag ctatgtctga atttagcgtg    4620
   tcacgtcaga ccgtgaatag agcacttaag gtctgcgggc attgaacttc cacgaggacg    4680
   ccgaaagctt cccagtaaat gtgccatctc gtaggcagaa aacggttccc ccgtagggtc    4740
   tctctcttgg cctcctttct aggtcgggct gattgctctt gaagctctct aggggggctc     4800
   acaccatagg cagataacgt tccccaccgg ctcgcctcgt aagcgcacaa ggactgctcc    4860
   caaagatctt caaagccact gccgcgactg ccttcgcgaa gccttgcccc gcggaaattt    4920
   cctccaccga gttcgtgcac acccctatgc caagcttctt tcaccctaaa ttcgagagat    4980
   tggattctta ccgtggaaat tcttcgcaaa aatcgtcccc tgatcgccct tgcgacgttg    5040
   gcgtcggtgc cgctggttgc gcttggcttg accgacttga tcagcggccg c             5091
<210> 10
   <211> 33
   <212> DNA
   <213> Künstliche Sequenz
<400> 10
   gagagcggcc gccgatcctt tttaacccat cac 33
<210> 11
   <211> 32
   <212> DNA
   <213> Künstliche Sequenz
<400> 11
   aggagcggcc gccatcggca ttttcttttg cg 32
<210> 12
   <211> 4323
   <212> DNA
   <213> Künstliche Sequenz
<400> 12
   tcgagaggcc tgacgtcggg cccggtacca cgcgtcatat gactagttcg gacctaggga      60
   tatcgtcgac atcgatgctc ttctgcgtta attaacaatt gggatcctct agacccggga     120
   tttaaatcgc tagcgggctg ctaaaggaag cggaacacgt agaaagccag tccgcagaaa     180
   cggtgctgac cccggatgaa tgtcagctac tgggctatct ggacaaggga aaacgcaagc     240
   gcaaagagaa agcaggtagc ttgcagtggg cttacatggc gatagctaga ctgggcggtt     300
   ttatggacag caagcgaacc ggaattgcca gctggggcgc cctctggtaa ggttgggaag     360
   ccctgcaaag taaactggat ggctttcttg ccgccaagga tctgatggcg caggggatca     420
   agatctgatc aagagacagg atgaggatcg tttcgcatga ttgaacaaga tggattgcac     480
   gcaggttctc cggccgcttg ggtggagagg ctattcggct atgactgggc acaacagaca     540
   atcggctgct ctgatgccgc cgtgttccgg ctgtcagcgc aggggcgccc ggttcttttt     600
   gtcaagaccg acctgtccgg tgccctgaat gaactgcagg acgaggcagc gcggctatcg     660
   tggctggcca cgacgggcgt tccttgcgca gctgtgctcg acgttgtcac tgaagcggga     720
   agggactggc tgctattggg cgaagtgccg gggcaggatc tcctgtcatc tcaccttgct     780
   cctgccgaga aagtatccat catggctgat gcaatgcggc ggctgcatac gcttgatccg     840
   gctacctgcc cattcgacca ccaagcgaaa catcgcatcg agcgagcacg tactcggatg     900
   gaagccggtc ttgtcgatca ggatgatctg gacgaagagc atcaggggct cgcgccagcc     960
   gaactgttcg ccaggctcaa ggcgcgcatg cccgacggcg aggatctcgt cgtgacccat    1020
   ggcgatgcct gcttgccgaa tatcatggtg gaaaatggcc gcttttctgg attcatcgac    1080
   tgtggccggc tgggtgtggc ggaccgctat caggacatag cgttggctac ccgtgatatt    1140
   gctgaagagc ttggcggcga atgggctgac cgcttcctcg tgctttacgg tatcgccgct    1200
   cccgattcgc agcgcatcgc cttctatcgc cttcttgacg agttcttctg agcgggactc    1260
   tggggttcga aatgaccgac caagcgacgc ccaacctgcc atcacgagat ttcgattcca    1320
   ccgccgcctt ctatgaaagg ttgggcttcg gaatcgtttt ccgggacgcc ggctggatga    1380
   tcctccagcg cggggatctc atgctggagt tcttcgccca cgctagcggc gcgccggccg    1440
   gcccggtgtg aaataccgca cagatgcgta aggagaaaat accgcatcag gcgctcttcc 1500
   gcttcctcgc tcactgactc gctgcgctcg gtcgttcggc tgcggcgagc ggtatcagct 1560
   cactcaaagg cggtaatacg gttatccaca gaatcagggg ataacgcagg aaagaacatg 1620
   tgagcaaaag gccagcaaaa ggccaggaac cgtaaaaagg ccgcgttgct ggcgtttttc 1680
   cataggctcc gcccccctga cgagcatcac aaaaatcgac gctcaagtca gaggtggcga 1740
   aacccgacag gactataaag ataccaggcg tttccccctg gaagctccct cgtgcgctct 1800
   cctgttccga ccctgccgct taccggatac ctgtccgcct ttctcccttc gggaagcgtg 1860
   gcgctttctc atagctcacg ctgtaggtat ctcagttcgg tgtaggtcgt tcgctccaag 1920
   ctgggctgtg tgcacgaacc ccccgttcag cccgaccgct gcgccttatc cggtaactat 1980
   cgtcttgagt ccaacccggt aagacacgac ttatcgccac tggcagcagc cactggtaac 2040
   aggattagca gagcgaggta tgtaggcggt gctacagagt tcttgaagtg gtggcctaac 2100
   tacggctaca ctagaaggac agtatttggt atctgcgctc tgctgaagcc agttaccttc 2160
   ggaaaaagag ttggtagctc ttgatccggc aaacaaacca ccgctggtag cggtggtttt 2220
   tttgtttgca agcagcagat tacgcgcaga aaaaaaggat ctcaagaaga tcctttgatc 2280
   ttttctacgg ggtctgacgc tcagtggaac gaaaactcac gttaagggat tttggtcatg 2340
   agattatcaa aaaggatctt cacctagatc cttttaaagg ccggccgcgg ccgccatcgg 2400
   cattttcttt tgcgttttta tttgttaact gttaattgtc cttgttcaag gatgctgtct 2460
   ttgacaacag atgttttctt gcctttgatg ttcagcagga agctcggcgc aaacgttgat 2520
   tgtttgtctg cgtagaatcc tctgtttgtc atatagcttg taatcacgac attgtttcct 2580
   ttcgcttgag gtacagcgaa gtgtgagtaa gtaaaggtta catcgttagg atcaagatcc 2640
   atttttaaca caaggccagt tttgttcagc ggcttgtatg ggccagttaa agaattagaa 2700
   acataaccaa gcatgtaaat atcgttagac gtaatgccgt caatcgtcat ttttgatccg 2760
   cgggagtcag tgaacaggta ccatttgccg ttcattttaa agacgttcgc gcgttcaatt 2820
   tcatctgtta ctgtgttaga tgcaatcagc ggtttcatca cttttttcag tgtgtaatca 2880
   tcgtttagct caatcatacc gagagcgccg tttgctaact cagccgtgcg ttttttatcg 2940
   ctttgcagaa gtttttgact ttcttgacgg aagaatgatg tgcttttgcc atagtatgct 3000
   ttgttaaata aagattcttc gccttggtag ccatcttcag ttccagtgtt tgcttcaaat 3060
   actaagtatt tgtggccttt atcttctacg tagtgaggat ctctcagcgt atggttgtcg 3120
   cctgagctgt agttgccttc atcgatgaac tgctgtacat tttgatacgt ttttccgtca 3180
   ccgtcaaaga ttgatttata atcctctaca ccgttgatgt tcaaagagct gtctgatgct 3240
   gatacgttaa cttgtgcagt tgtcagtgtt tgtttgccgt aatgtttacc ggagaaatca 3300
   gtgtagaata aacggatttt tccgtcagat gtaaatgtgg ctgaacctga ccattcttgt 3360
   gtttggtctt ttaggataga atcatttgca tcgaatttgt cgctgtcttt aaagacgcgg 3420
   ccagcgtttt tccagctgtc aatagaagtt tcgccgactt tttgatagaa catgtaaatc 3480
   gatgtgtcat ccgcattttt aggatctccg gctaatgcaa agacgatgtg gtagccgtga 3540
   tagtttgcga cagtgccgtc agcgttttgt aatggccagc tgtcccaaac gtccaggcct 3600
   tttgcagaag agatattttt aattgtggac gaatcaaatt cagaaacttg atatttttca 3660
   tttttttgct gttcagggat ttgcagcata tcatggcgtg taatatggga aatgccgtat 3720
   gtttccttat atggcttttg gttcgtttct ttcgcaaacg cttgagttgc gcctcctgcc 3780
   agcagtgcgg tagtaaaggt taatactgtt gcttgttttg caaacttttt gatgttcatc 3840
   gttcatgtct ccttttttat gtactgtgtt agcggtctgc ttcttccagc cctcctgttt 3900
   gaagatggca agttagttac gcacaataaa aaaagaccta aaatatgtaa ggggtgacgc 3960
   caaagtatac actttgccct ttacacattt taggtcttgc ctgctttatc agtaacaaac 4020
   ccgcgcgatt tacttttcga cctcattcta ttagactctc gtttggattg caactggtct 4080
   attttcctct tttgtttgat agaaaatcat aaaaggattt gcagactacg ggcctaaaga 4140
   actaaaaaat ctatctgttt cttttcattc tctgtatttt ttatagtttc tgttgcatgg 4200
   gcataaagtt gcctttttaa tcacaattca gaaaatatca taatatctca tttcactaaa 4260
   taatagtgaa cggcaggtat atgtgatggg ttaaaaagga tcggcggccg ctcgatttaa 4320
   atc 4323
<210> 13
   <211> 34
   <212> DNA
   <213> Künstliche Sequenz
<400> 13
   gagagcccgg gaagaagggc tgcgacctcc tcat 34
<210> 14
   <211> 34
   <212> DNA
   <213> Künstliche Sequenz
<400> 14
   ctctcacgcg tcatatgcag gtgaggtaac ccca 34
<210> 15
   <211> 6631
   <212> DNA
   <213> Künstliche Sequenz
<400> 15
   cgcgtcatat gcaggtgagg taaccccaaa agaggtaaaa cccgcgccac cgacttttca 60
   ggagcgggga cgcgggtttt tgccatgaat ccgaagatac tacatcagat ttttaggcca 120
   acttgagggc tgcgcgaagt tcatcaacgc ggtcgatagc ctcccaagga aggtccaaat 180
   cagtgcgacc aaagtggccg taggcagcag tgtcagcgta gatcggacga agcagatcaa 240
   gctcacggat aattgctgct ggacgcaggt caaagacctc caacacggca gcctgaatct 300
   gctcgtcgct caggccttcc ttgttggtgt caaaggtttc aacgtaaagt ccgactggct 360
   ttgcgcgtcc aatggcgtat gcaacctgaa cttcagcgcg atcagcaagg cctgctgcca 420
   cgatgttctt tgctacccaa cgcatggcgt atgcagcaga gcggtccacc ttgcttggat 480
   ccttaccgga gaatgctcca ccaccatggc gagccatgcc accgtaggta tccacgatga 540
   tcttgcggcc ggtcagaccc gcatcaccca tggggccacc cagaatgaag gaacctgaag 600
   ggttgatcaa cacggtgatc tcaccggttg ccagatcctc aatgcctgcg tctttgatta 660
   cccaatcaat gacgtgttcg cgcagttggg tttccaacca tgcacggtca acttctgggt 720
   cgtgctgggt ggagatgaca acggtatcca ggtggctagg gcggtcttgc gcatcgtatg 780
   cgaaggtgac ctgggttttt ccgtctggac gcaggtgagg aacgatgccc tctttacgaa 840
   cctgggtcag acgacgtgac agtcggtgcg ccaacgcgat aggaagaggc atgtactctt 900
   cggtttcgtt ggtggcgtag ccgaacatca ggccctggtc gccagcacct gcgcggtcgt 960
   cttcttcaac gtcgccgttg gtgcgggctt cgtcggagtt atccacgccg tcagcgattt 1020
   cctgggactg ctcaccgatg gatactgaga cgccacaggt gcgtccgtcg aatccaacct 1080
   cagaggagtt gaatccgatt tcgatgagct tgttgcggac taattgaggg atctctacgt 1140
   aagcgctggt acggacctcg ccaacaacat ggacgattcc ggtggtgacc acagtttcca 1200
   ctgcgacgcg cgactgcgga tctttttcga gcagcgcgtc caaaatggta tcggaaatag 1260
   catcacatat tttgtctgga tgtccctcag ttacagattc actggtgaac aaacggacgg 1320
   cggttggctg agccacaaat acccttcttt cgaagaagtt gagaataaat agtcttaaat 1380
   acaaaaaacc aatatagacc aagctgtcta aaactgcaat gtcagtggtc tagctggatt 1440
   tttctagact tcgcgatacg ccagtgccgc gtcccaaatt tgcgcagcaa cctcgatttt 1500
   gctgccgtgc tccacatcga ctaccccacc gtgagcatcc aaaatccagc cctcattgtg 1560
   cttttgccca aacactttgc ccatgcccac ctcattacac atgaggaggt cgcagccctt 1620
   cttcccggga tttaaatcgc tagcgggctg ctaaaggaag cggaacacgt agaaagccag 1680
   tccgcagaaa cggtgctgac cccggatgaa tgtcagctac tgggctatct ggacaaggga 1740
   aaacgcaagc gcaaagagaa agcaggtagc ttgcagtggg cttacatggc gatagctaga 1800
   ctgggcggtt ttatggacag caagcgaacc ggaattgcca gctggggcgc cctctggtaa 1860
   ggttgggaag ccctgcaaag taaactggat ggctttcttg ccgccaagga tctgatggcg 1920
   caggggatca agatctgatc aagagacagg atgaggatcg tttcgcatga ttgaacaaga 1980
   tggattgcac gcaggttctc cggccgcttg ggtggagagg ctattcggct atgactgggc 2040
   acaacagaca atcggctgct ctgatgccgc cgtgttccgg ctgtcagcgc aggggcgccc 2100
   ggttcttttt gtcaagaccg acctgtccgg tgccctgaat gaactgcagg acgaggcagc 2160
   gcggctatcg tggctggcca cgacgggcgt tccttgcgca gctgtgctcg acgttgtcac 2220
   tgaagcggga agggactggc tgctattggg cgaagtgccg gggcaggatc tcctgtcatc 2280
   tcaccttgct cctgccgaga aagtatccat catggctgat gcaatgcggc ggctgcatac 2340
   gcttgatccg gctacctgcc cattcgacca ccaagcgaaa catcgcatcg agcgagcacg 2400
   tactcggatg gaagccggtc ttgtcgatca ggatgatctg gacgaagagc atcaggggct 2460
   cgcgccagcc gaactgttcg ccaggctcaa ggcgcgcatg cccgacggcg aggatctcgt 2520
   cgtgacccat ggcgatgcct gcttgccgaa tatcatggtg gaaaatggcc gcttttctgg 2580
   attcatcgac tgtggccggc tgggtgtggc ggaccgctat caggacatag cgttggctac 2640
   ccgtgatatt gctgaagagc ttggcggcga atgggctgac cgcttcctcg tgctttacgg 2700
   tatcgccgct cccgattcgc agcgcatcgc cttctatcgc cttcttgacg agttcttctg 2760
   agcgggactc tggggttcga aatgaccgac caagcgacgc ccaacctgcc atcacgagat 2820
   ttcgattcca ccgccgcctt ctatgaaagg ttgggcttcg gaatcgtttt ccgggacgcc 2880
   ggctggatga tcctccagcg cggggatctc atgctggagt tcttcgccca cgctagcggc 2940
   gcgccggccg gcccggtgtg aaataccgca cagatgcgta aggagaaaat accgcatcag 3000
   gcgctcttcc gcttcctcgc tcactgactc gctgcgctcg gtcgttcggc tgcggcgagc 3060
   ggtatcagct cactcaaagg cggtaatacg gttatccaca gaatcagggg ataacgcagg 3120
   aaagaacatg tgagcaaaag gccagcaaaa ggccaggaac cgtaaaaagg ccgcgttgct 3180
   ggcgtttttc cataggctcc gcccccctga cgagcatcac aaaaatcgac gctcaagtca 3240
   gaggtggcga aacccgacag gactataaag ataccaggcg tttccccctg gaagctccct 3300
   cgtgcgctct cctgttccga ccctgccgct taccggatac ctgtccgcct ttctcccttc 3360
   gggaagcgtg gcgctttctc atagctcacg ctgtaggtat ctcagttcgg tgtaggtcgt 3420
   tcgctccaag ctgggctgtg tgcacgaacc ccccgttcag cccgaccgct gcgccttatc 3480
   cggtaactat cgtcttgagt ccaacccggt aagacacgac ttatcgccac tggcagcagc 3540
   cactggtaac aggattagca gagcgaggta tgtaggcggt gctacagagt tcttgaagtg 3600
   gtggcctaac tacggctaca ctagaaggac agtatttggt atctgcgctc tgctgaagcc 3660
   agttaccttc ggaaaaagag ttggtagctc ttgatccggc aaacaaacca ccgctggtag 3720
   cggtggtttt tttgtttgca agcagcagat tacgcgcaga aaaaaaggat ctcaagaaga 3780
   tcctttgatc ttttctacgg ggtctgacgc tcagtggaac gaaaactcac gttaagggat 3840
   tttggtcatg agattatcaa aaaggatctt cacctagatc cttttaaagg ccggccgcgg 3900
   ccgcgcaaag tcccgcttcg tgaaaatttt cgtgccgcgt gattttccgc caaaaacttt 3960
   aacgaacgtt cgttataatg gtgtcatgac cttcacgacg aagtactaaa attggcccga 4020
   atcatcagct atggatctct ctgatgtcgc gctggagtcc gacgcgctcg atgctgccgt 4080
   cgatttaaaa acggtgatcg gatttttccg agctctcgat acgacggacg cgccagcatc 4140
   acgagactgg gccagtgccg cgagcgacct agaaactctc gtggcggatc ttgaggagct 4200
   ggctgacgag ctgcgtgctc ggccagcgcc aggaggacgc acagtagtgg aggatgcaat 4260
   cagttgcgcc tactgcggtg gcctgattcc tccccggcct gacccgcgag gacggcgcgc 4320
   aaaatattgc tcagatgcgt gtcgtgccgc agccagccgc gagcgcgcca acaaacgcca 4380
   cgccgaggag ctggaggcgg ctaggtcgca aatggcgctg gaagtgcgtc ccccgagcga 4440
   aattttggcc atggtcgtca cagagctgga agcggcagcg agaattatcg cgatcgtggc 4500
   ggtgcccgca ggcatgacaa acatcgtaaa tgccgcgttt cgtgtgccgt ggccgcccag 4560
   gacgtgtcag cgccgccacc acctgcaccg aatcggcagc agcgtcgcgc gtcgaaaaag 4620
   cgcacaggcg gcaagaagcg ataagctgca cgaatacctg aaaaatgttg aacgccccgt 4680
   gagcggtaac tcacagggcg tcggctaacc cccagtccaa acctgggaga aagcgctcaa 4740
   aaatgactct agcggattca cgagacattg acacaccggc ctggaaattt tccgctgatc 4800
   tgttcgacac ccatcccgag ctcgcgctgc gatcacgtgg ctggacgagc gaagaccgcc 4860
   gcgaattcct cgctcacctg ggcagagaaa atttccaggg cagcaagacc cgcgacttcg 4920
   ccagcgcttg gatcaaagac ccggacacgg agaaacacag ccgaagttat accgagttgg 4980
   ttcaaaatcg cttgcccggt gccagtatgt tgctctgacg cacgcgcagc acgcagccgt 5040
   gcttgtcctg gacattgatg tgccgagcca ccaggccggc gggaaaatcg agcacgtaaa 5100
   ccccgaggtc tacgcgattt tggagcgctg ggcacgcctg gaaaaagcgc cagcttggat 5160
   cggcgtgaat ccactgagcg ggaaatgcca gctcatctgg ctcattgatc cggtgtatgc 5220
   cgcagcaggc atgagcagcc cgaatatgcg cctgctggct gcaacgaccg aggaaatgac 5280
   ccgcgttttc ggcgctgacc aggctttttc acataggctg agccgtggcc actgcactct 5340
   ccgacgatcc cagccgtacc gctggcatgc ccagcacaat cgcgtggatc gcctagctga 5400
   tcttatggag gttgctcgca tgatctcagg cacagaaaaa cctaaaaaac gctatgagca 5460
   ggagttttct agcggacggg cacgtatcga agcggcaaga aaagccactg cggaagcaaa 5520
   agcacttgcc acgcttgaag caagcctgcc gagcgccgct gaagcgtctg gagagctgat 5580
   cgacggcgtc cgtgtcctct ggactgctcc agggcgtgcc gcccgtgatg agacggcttt 5640
   tcgccacgct ttgactgtgg gataccagtt aaaagcggct ggtgagcgcc taaaagacac 5700
   caagggtcat cgagcctacg agcgtgccta caccgtcgct caggcggtcg gaggaggccg 5760
   tgagcctgat ctgccgccgg actgtgaccg ccagacggat tggccgcgac gtgtgcgcgg 5820
   ctacgtcgct aaaggccagc cagtcgtccc tgctcgtcag acagagacgc agagccagcc 5880
   gaggcgaaaa gctctggcca ctatgggaag acgtggcggt aaaaaggccg cagaacgctg 5940
   gaaagaccca aacagtgagt acgcccgagc acagcgagaa aaactagcta agtccagtca 6000
   acgacaagct aggaaagcta aaggaaatcg cttgaccatt gcaggttggt ttatgactgt 6060
   tgagggagag actggctcgt ggccgacaat caatgaagct atgtctgaat ttagcgtgtc 6120
   acgtcagacc gtgaatagag cacttaaggt ctgcgggcat tgaacttcca cgaggacgcc 6180
   gaaagcttcc cagtaaatgt gccatctcgt aggcagaaaa cggttccccc gtagggtctc 6240
   tctcttggcc tcctttctag gtcgggctga ttgctcttga agctctctag gggggctcac 6300
   accataggca gataacgttc cccaccggct cgcctcgtaa gcgcacaagg actgctccca 6360
   aagatcttca aagccactgc cgcgactgcc ttcgcgaagc cttgccccgc ggaaatttcc 6420
   tccaccgagt tcgtgcacac ccctatgcca agcttctttc accctaaatt cgagagattg 6480
   gattcttacc gtggaaattc ttcgcaaaaa tcgtcccctg atcgcccttg cgacgttggc 6540
   gtcggtgccg ctggttgcgc ttggcttgac cgacttgatc agcggccgct cgatttaaat 6600
   ctcgagaggc ctgacgtcgg gcccggtacc a 6631
<210> 16
   <211> 407
   <212> PRT
   <213> Corynebacterium glutamicum
<400> 16
   Val Ala Gln Pro Thr Ala Val Arg Leu Phe Thr Ser Glu Ser Val Thr
   1 5 10 15
   Glu Gly His Pro Asp Lys Ile Cys Asp Ala Ile Ser Asp Thr Ile Leu
   20 25 30
   Asp Ala Leu Leu Glu Lys Asp Pro Gln Ser Arg Val Ala Val Glu Thr
   35 40 45
   Val Val Thr Thr Gly Ile Val His Val Val Gly Glu Val Arg Thr Ser
   50 55 60
   Ala Tyr Val Glu Ile Pro Gln Leu Val Arg Asn Lys Leu Ile Glu Ile
   65 70 75 80
   Gly Phe Asn Ser Ser Glu Val Gly Phe Asp Gly Arg Thr Cys Gly Val
   85 90 95
   Ser Val Ser Ile Gly Glu Gln Ser Gln Glu Ile Ala Asp Gly Val Asp
   100 105 110
   Asn Ser Asp Glu Ala Arg Thr Asn Gly Asp Val Glu Glu Asp Asp Arg
   115 120 125
   Ala Gly Ala Gly Asp Gln Gly Leu Met Phe Gly Tyr Ala Thr Asn Glu
   130 135 140
   Thr Glu Glu Tyr Met Pro Leu Pro Ile Ala Leu Ala His Arg Leu Ser
   145 150 155 160
   Arg Arg Leu Thr Gln Val Arg Lys Glu Gly Ile Val Pro His Leu Arg
                   165                 170                 175
   Pro Asp Gly Lys Thr Gln Val Thr Phe Ala Tyr Asp Ala Gln Asp Arg
               180                 185                 190
   Pro Ser His Leu Asp Thr Val Val Ile Ser Thr Gln His Asp Pro Glu
           195                 200                 205
   Val Asp Arg Ala Trp Leu Glu Thr Gln Leu Arg Glu His Val Ile Asp
       210                 215                 220
   Trp Val Ile Lys Asp Ala Gly Ile Glu Asp Leu Ala Thr Gly Glu Ile
   225                 230                 235                 240
   Thr Val Leu Ile Asn Pro Ser Gly Ser Phe Ile Leu Gly Gly Pro Met
                   245                 250                 255
   Gly Asp Ala Gly Leu Thr Gly Arg Lys Ile Ile Val Asp Thr Tyr Gly
               260                 265                 270
   Gly Met Ala Arg His Gly Gly Gly Ala Phe Ser Gly Lys Asp Pro Ser
           275                 280                 285
   Lys Val Asp Arg Ser Ala Ala Tyr Ala Met Arg Trp Val Ala Lys Asn
       290                 295                 300
   Ile Val Ala Ala Gly Leu Ala Asp Arg Ala Glu Val Gln Val Ala Tyr
   305                 310                 315                 320
   Ala Ile Gly Arg Ala Lys Pro Val Gly Leu Tyr Val Glu Thr Phe Asp
                   325                 330                 335
   Thr Asn Lys Glu Gly Leu Ser Asp Glu Gln Ile Gln Ala Ala Val Leu
               340                 345                 350
   Glu Val Phe Asp Leu Arg Pro Ala Ala Ile Ile Arg Glu Leu Asp Leu
           355                 360                 365
   Leu Arg Pro Ile Tyr Ala Asp Thr Ala Ala Tyr Gly His Phe Gly Arg
       370                 375                 380
   Thr Asp Leu Asp Leu Pro Trp Glu Ala Ile Asp Arg Val Asp Glu Leu
   385                 390                 395                 400
   Arg Ala Ala Leu Lys Leu Ala
   405
<210> 17
   <211> 38
   <212> DNA
   <213> Künstliche Sequenz
<400> 17
   gattcgacgg acgcaccgct ggcgtctcag tatccatc 38
<210> 18
   <211> 38
   <212> DNA
   <213> Künstliche Sequenz
<400> 18
   gatggatact gagacgccag cggtgcgtcc gtcgaatc 38
<210> 19
   <211> 6631
   <212> DNA
   <213> Künstliche Sequenz
<400> 19
   cgcgtcatat gcaggtgagg taaccccaaa agaggtaaaa cccgcgccac cgacttttca 60
   ggagcgggga cgcgggtttt tgccatgaat ccgaagatac tacatcagat ttttaggcca 120
   acttgagggc tgcgcgaagt tcatcaacgc ggtcgatagc ctcccaagga aggtccaaat 180
   cagtgcgacc aaagtggccg taggcagcag tgtcagcgta gatcggacga agcagatcaa 240
   gctcacggat aattgctgct ggacgcaggt caaagacctc caacacggca gcctgaatct 300
   gctcgtcgct caggccttcc ttgttggtgt caaaggtttc aacgtaaagt ccgactggct 360
   ttgcgcgtcc aatggcgtat gcaacctgaa cttcagcgcg atcagcaagg cctgctgcca 420
   cgatgttctt tgctacccaa cgcatggcgt atgcagcaga gcggtccacc ttgcttggat 480
   ccttaccgga gaatgctcca ccaccatggc gagccatgcc accgtaggta tccacgatga 540
   tcttgcggcc ggtcagaccc gcatcaccca tggggccacc cagaatgaag gaacctgaag 600
   ggttgatcaa cacggtgatc tcaccggttg ccagatcctc aatgcctgcg tctttgatta 660
   cccaatcaat gacgtgttcg cgcagttggg tttccaacca tgcacggtca acttctgggt 720
   cgtgctgggt ggagatgaca acggtatcca ggtggctagg gcggtcttgc gcatcgtatg 780
   cgaaggtgac ctgggttttt ccgtctggac gcaggtgagg aacgatgccc tctttacgaa 840
   cctgggtcag acgacgtgac agtcggtgcg ccaacgcgat aggaagaggc atgtactctt 900
   cggtttcgtt ggtggcgtag ccgaacatca ggccctggtc gccagcacct gcgcggtcgt 960
   cttcttcaac gtcgccgttg gtgcgggctt cgtcggagtt atccacgccg tcagcgattt 1020
   cctgggactg ctcaccgatg gatactgaga cgccagcggt gcgtccgtcg aatccaacct 1080
   cagaggagtt gaatccgatt tcgatgagct tgttgcggac taattgaggg atctctacgt 1140
   aagcgctggt acggacctcg ccaacaacat ggacgattcc ggtggtgacc acagtttcca 1200
   ctgcgacgcg cgactgcgga tctttttcga gcagcgcgtc caaaatggta tcggaaatag 1260
   catcacatat tttgtctgga tgtccctcag ttacagattc actggtgaac aaacggacgg 1320
   cggttggctg agccacaaat acccttcttt cgaagaagtt gagaataaat agtcttaaat 1380
   acaaaaaacc aatatagacc aagctgtcta aaactgcaat gtcagtggtc tagctggatt 1440
   tttctagact tcgcgatacg ccagtgccgc gtcccaaatt tgcgcagcaa cctcgatttt 1500
   gctgccgtgc tccacatcga ctaccccacc gtgagcatcc aaaatccagc cctcattgtg 1560
   cttttgccca aacactttgc ccatgcccac ctcattacac atgaggaggt cgcagccctt 1620
   cttcccggga tttaaatcgc tagcgggctg ctaaaggaag cggaacacgt agaaagccag 1680
   tccgcagaaa cggtgctgac cccggatgaa tgtcagctac tgggctatct ggacaaggga 1740
   aaacgcaagc gcaaagagaa agcaggtagc ttgcagtggg cttacatggc gatagctaga 1800
   ctgggcggtt ttatggacag caagcgaacc ggaattgcca gctggggcgc cctctggtaa 1860
   ggttgggaag ccctgcaaag taaactggat ggctttcttg ccgccaagga tctgatggcg 1920
   caggggatca agatctgatc aagagacagg atgaggatcg tttcgcatga ttgaacaaga 1980
   tggattgcac gcaggttctc cggccgcttg ggtggagagg ctattcggct atgactgggc 2040
   acaacagaca atcggctgct ctgatgccgc cgtgttccgg ctgtcagcgc aggggcgccc 2100
   ggttcttttt gtcaagaccg acctgtccgg tgccctgaat gaactgcagg acgaggcagc 2160
   gcggctatcg tggctggcca cgacgggcgt tccttgcgca gctgtgctcg acgttgtcac 2220
   tgaagcggga agggactggc tgctattggg cgaagtgccg gggcaggatc tcctgtcatc 2280
   tcaccttgct cctgccgaga aagtatccat catggctgat gcaatgcggc ggctgcatac 2340
   gcttgatccg gctacctgcc cattcgacca ccaagcgaaa catcgcatcg agcgagcacg 2400
   tactcggatg gaagccggtc ttgtcgatca ggatgatctg gacgaagagc atcaggggct 2460
   cgcgccagcc gaactgttcg ccaggctcaa ggcgcgcatg cccgacggcg aggatctcgt 2520
   cgtgacccat ggcgatgcct gcttgccgaa tatcatggtg gaaaatggcc gcttttctgg 2580
   attcatcgac tgtggccggc tgggtgtggc ggaccgctat caggacatag cgttggctac 2640
   ccgtgatatt gctgaagagc ttggcggcga atgggctgac cgcttcctcg tgctttacgg 2700
   tatcgccgct cccgattcgc agcgcatcgc cttctatcgc cttcttgacg agttcttctg 2760
   agcgggactc tggggttcga aatgaccgac caagcgacgc ccaacctgcc atcacgagat 2820
   ttcgattcca ccgccgcctt ctatgaaagg ttgggcttcg gaatcgtttt ccgggacgcc 2880
   ggctggatga tcctccagcg cggggatctc atgctggagt tcttcgccca cgctagcggc 2940
   gcgccggccg gcccggtgtg aaataccgca cagatgcgta aggagaaaat accgcatcag 3000
   gcgctcttcc gcttcctcgc tcactgactc gctgcgctcg gtcgttcggc tgcggcgagc 3060
   ggtatcagct cactcaaagg cggtaatacg gttatccaca gaatcagggg ataacgcagg 3120
   aaagaacatg tgagcaaaag gccagcaaaa ggccaggaac cgtaaaaagg ccgcgttgct 3180
   ggcgtttttc cataggctcc gcccccctga cgagcatcac aaaaatcgac gctcaagtca 3240
   gaggtggcga aacccgacag gactataaag ataccaggcg tttccccctg gaagctccct 3300
   cgtgcgctct cctgttccga ccctgccgct taccggatac ctgtccgcct ttctcccttc 3360
   gggaagcgtg gcgctttctc atagctcacg ctgtaggtat ctcagttcgg tgtaggtcgt 3420
   tcgctccaag ctgggctgtg tgcacgaacc ccccgttcag cccgaccgct gcgccttatc 3480
   cggtaactat cgtcttgagt ccaacccggt aagacacgac ttatcgccac tggcagcagc 3540
   cactggtaac aggattagca gagcgaggta tgtaggcggt gctacagagt tcttgaagtg 3600
   gtggcctaac tacggctaca ctagaaggac agtatttggt atctgcgctc tgctgaagcc 3660
   agttaccttc ggaaaaagag ttggtagctc ttgatccggc aaacaaacca ccgctggtag 3720
   cggtggtttt tttgtttgca agcagcagat tacgcgcaga aaaaaaggat ctcaagaaga 3780
   tcctttgatc ttttctacgg ggtctgacgc tcagtggaac gaaaactcac gttaagggat 3840
   tttggtcatg agattatcaa aaaggatctt cacctagatc cttttaaagg ccggccgcgg 3900
   ccgcgcaaag tcccgcttcg tgaaaatttt cgtgccgcgt gattttccgc caaaaacttt 3960
   aacgaacgtt cgttataatg gtgtcatgac cttcacgacg aagtactaaa attggcccga 4020
   atcatcagct atggatctct ctgatgtcgc gctggagtcc gacgcgctcg atgctgccgt 4080
   cgatttaaaa acggtgatcg gatttttccg agctctcgat acgacggacg cgccagcatc 4140
   acgagactgg gccagtgccg cgagcgacct agaaactctc gtggcggatc ttgaggagct 4200
   ggctgacgag ctgcgtgctc ggccagcgcc aggaggacgc acagtagtgg aggatgcaat 4260
   cagttgcgcc tactgcggtg gcctgattcc tccccggcct gacccgcgag gacggcgcgc 4320
   aaaatattgc tcagatgcgt gtcgtgccgc agccagccgc gagcgcgcca acaaacgcca 4380
   cgccgaggag ctggaggcgg ctaggtcgca aatggcgctg gaagtgcgtc ccccgagcga 4440
   aattttggcc atggtcgtca cagagctgga agcggcagcg agaattatcg cgatcgtggc 4500
   ggtgcccgca ggcatgacaa acatcgtaaa tgccgcgttt cgtgtgccgt ggccgcccag 4560
   gacgtgtcag cgccgccacc acctgcaccg aatcggcagc agcgtcgcgc gtcgaaaaag 4620
   cgcacaggcg gcaagaagcg ataagctgca cgaatacctg aaaaatgttg aacgccccgt 4680
   gagcggtaac tcacagggcg tcggctaacc cccagtccaa acctgggaga aagcgctcaa 4740
   aaatgactct agcggattca cgagacattg acacaccggc ctggaaattt tccgctgatc 4800
   tgttcgacac ccatcccgag ctcgcgctgc gatcacgtgg ctggacgagc gaagaccgcc 4860
   gcgaattcct cgctcacctg ggcagagaaa atttccaggg cagcaagacc cgcgacttcg 4920
   ccagcgcttg gatcaaagac ccggacacgg agaaacacag ccgaagttat accgagttgg 4980
   ttcaaaatcg cttgcccggt gccagtatgt tgctctgacg cacgcgcagc acgcagccgt 5040
   gcttgtcctg gacattgatg tgccgagcca ccaggccggc gggaaaatcg agcacgtaaa 5100
   ccccgaggtc tacgcgattt tggagcgctg ggcacgcctg gaaaaagcgc cagcttggat 5160
   cggcgtgaat ccactgagcg ggaaatgcca gctcatctgg ctcattgatc cggtgtatgc 5220
   cgcagcaggc atgagcagcc cgaatatgcg cctgctggct gcaacgaccg aggaaatgac 5280
   ccgcgttttc ggcgctgacc aggctttttc acataggctg agccgtggcc actgcactct 5340
   ccgacgatcc cagccgtacc gctggcatgc ccagcacaat cgcgtggatc gcctagctga 5400
   tcttatggag gttgctcgca tgatctcagg cacagaaaaa cctaaaaaac gctatgagca 5460
   ggagttttct agcggacggg cacgtatcga agcggcaaga aaagccactg cggaagcaaa 5520
   agcacttgcc acgcttgaag caagcctgcc gagcgccgct gaagcgtctg gagagctgat 5580
   cgacggcgtc cgtgtcctct ggactgctcc agggcgtgcc gcccgtgatg agacggcttt 5640
   tcgccacgct ttgactgtgg gataccagtt aaaagcggct ggtgagcgcc taaaagacac 5700
   caagggtcat cgagcctacg agcgtgccta caccgtcgct caggcggtcg gaggaggccg 5760
   tgagcctgat ctgccgccgg actgtgaccg ccagacggat tggccgcgac gtgtgcgcgg   5820
   ctacgtcgct aaaggccagc cagtcgtccc tgctcgtcag acagagacgc agagccagcc   5880
   gaggcgaaaa gctctggcca ctatgggaag acgtggcggt aaaaaggccg cagaacgctg   5940
   gaaagaccca aacagtgagt acgcccgagc acagcgagaa aaactagcta agtccagtca   6000
   acgacaagct aggaaagcta aaggaaatcg cttgaccatt gcaggttggt ttatgactgt   6060
   tgagggagag actggctcgt ggccgacaat caatgaagct atgtctgaat ttagcgtgtc   6120
   acgtcagacc gtgaatagag cacttaaggt ctgcgggcat tgaacttcca cgaggacgcc   6180
   gaaagcttcc cagtaaatgt gccatctcgt aggcagaaaa cggttccccc gtagggtctc   6240
   tctcttggcc tcctttctag gtcgggctga ttgctcttga agctctctag gggggctcac   6300
   accataggca gataacgttc cccaccggct cgcctcgtaa gcgcacaagg actgctccca   6360
   aagatcttca aagccactgc cgcgactgcc ttcgcgaagc cttgccccgc ggaaatttcc   6420
   tccaccgagt tcgtgcacac ccctatgcca agcttctttc accctaaatt cgagagattg   6480
   gattcttacc gtggaaattc ttcgcaaaaa tcgtcccctg atcgcccttg cgacgttggc   6540
   gtcggtgccg ctggttgcgc ttggcttgac cgacttgatc agcggccgct cgatttaaat   6600
   ctcgagaggc ctgacgtcgg gcccggtacc a                                  6631
<210> 20
   <211> 5863
   <212> DNA
   <213> Künstliche Sequenz
<400> 20
   tcgagaggcc tgacgtcggg cccggtacca cgcgtcatat gcaggtgagg taaccccaaa     60
   agaggtaaaa cccgcgccac cgacttttca ggagcgggga cgcgggtttt tgccatgaat    120
   ccgaagatac tacatcagat ttttaggcca acttgagggc tgcgcgaagt tcatcaacgc    180
   ggtcgatagc ctcccaagga aggtccaaat cagtgcgacc aaagtggccg taggcagcag    240
   tgtcagcgta gatcggacga agcagatcaa gctcacggat aattgctgct ggacgcaggt    300
   caaagacctc caacacggca gcctgaatct gctcgtcgct caggccttcc ttgttggtgt    360
   caaaggtttc aacgtaaagt ccgactggct ttgcgcgtcc aatggcgtat gcaacctgaa    420
   cttcagcgcg atcagcaagg cctgctgcca cgatgttctt tgctacccaa cgcatggcgt    480
   atgcagcaga gcggtccacc ttgcttggat ccttaccgga gaatgctcca ccaccatggc    540
   gagccatgcc accgtaggta tccacgatga tcttgcggcc ggtcagaccc gcatcaccca    600
   tggggccacc cagaatgaag gaacctgaag ggttgatcaa cacggtgatc tcaccggttg    660
   ccagatcctc aatgcctgcg tctttgatta cccaatcaat gacgtgttcg cgcagttggg    720
   tttccaacca tgcacggtca acttctgggt cgtgctgggt ggagatgaca acggtatcca    780
   ggtggctagg gcggtcttgc gcatcgtatg cgaaggtgac ctgggttttt ccgtctggac    840
   gcaggtgagg aacgatgccc tctttacgaa cctgggtcag acgacgtgac agtcggtgcg    900
   ccaacgcgat aggaagaggc atgtactctt cggtttcgtt ggtggcgtag ccgaacatca    960
   ggccctggtc gccagcacct gcgcggtcgt cttcttcaac gtcgccgttg gtgcgggctt   1020
   cgtcggagtt atccacgccg tcagcgattt cctgggactg ctcaccgatg gatactgaga   1080
   cgccagcggt gcgtccgtcg aatccaacct cagaggagtt gaatccgatt tcgatgagct   1140
   tgttgcggac taattgaggg atctctacgt aagcgctggt acggacctcg ccaacaacat   1200
   ggacgattcc ggtggtgacc acagtttcca ctgcgacgcg cgactgcgga tctttttcga   1260
   gcagcgcgtc caaaatggta tcggaaatag catcacatat tttgtctgga tgtccctcag   1320
   ttacagattc actggtgaac aaacggacgg cggttggctg agccacaaat acccttcttt   1380
   cgaagaagtt gagaataaat agtcttaaat acaaaaaacc aatatagacc aagctgtcta   1440
   aaactgcaat gtcagtggtc tagctggatt tttctagact tcgcgatacg ccagtgccgc   1500
   gtcccaaatt tgcgcagcaa cctcgatttt gctgccgtgc tccacatcga ctaccccacc   1560
   gtgagcatcc aaaatccagc cctcattgtg cttttgccca aacactttgc ccatgcccac   1620
   ctcattacac atgaggaggt cgcagccctt cttcccggga tttaaatcgc tagcgggctg   1680
   ctaaaggaag cggaacacgt agaaagccag tccgcagaaa cggtgctgac cccggatgaa   1740
   tgtcagctac tgggctatct ggacaaggga aaacgcaagc gcaaagagaa agcaggtagc   1800
   ttgcagtggg cttacatggc gatagctaga ctgggcggtt ttatggacag caagcgaacc   1860
   ggaattgcca gctggggcgc cctctggtaa ggttgggaag ccctgcaaag taaactggat   1920
   ggctttcttg ccgccaagga tctgatggcg caggggatca agatctgatc aagagacagg   1980
   atgaggatcg tttcgcatga ttgaacaaga tggattgcac gcaggttctc cggccgcttg   2040
   ggtggagagg ctattcggct atgactgggc acaacagaca atcggctgct ctgatgccgc   2100
   cgtgttccgg ctgtcagcgc aggggcgccc ggttcttttt gtcaagaccg acctgtccgg   2160
   tgccctgaat gaactgcagg acgaggcagc gcggctatcg tggctggcca cgacgggcgt   2220
   tccttgcgca gctgtgctcg acgttgtcac tgaagcggga agggactggc tgctattggg   2280
   cgaagtgccg gggcaggatc tcctgtcatc tcaccttgct cctgccgaga aagtatccat 2340
   catggctgat gcaatgcggc ggctgcatac gcttgatccg gctacctgcc cattcgacca 2400
   ccaagcgaaa catcgcatcg agcgagcacg tactcggatg gaagccggtc ttgtcgatca 2460
   ggatgatctg gacgaagagc atcaggggct cgcgccagcc gaactgttcg ccaggctcaa 2520
   ggcgcgcatg cccgacggcg aggatctcgt cgtgacccat ggcgatgcct gcttgccgaa 2580
   tatcatggtg gaaaatggcc gcttttctgg attcatcgac tgtggccggc tgggtgtggc 2640
   ggaccgctat caggacatag cgttggctac ccgtgatatt gctgaagagc ttggcggcga 2700
   atgggctgac cgcttcctcg tgctttacgg tatcgccgct cccgattcgc agcgcatcgc 2760
   cttctatcgc cttcttgacg agttcttctg agcgggactc tggggttcga aatgaccgac 2820
   caagcgacgc ccaacctgcc atcacgagat ttcgattcca ccgccgcctt ctatgaaagg 2880
   ttgggcttcg gaatcgtttt ccgggacgcc ggctggatga tcctccagcg cggggatctc 2940
   atgctggagt tcttcgccca cgctagcggc gcgccggccg gcccggtgtg aaataccgca 3000
   cagatgcgta aggagaaaat accgcatcag gcgctcttcc gcttcctcgc tcactgactc 3060
   gctgcgctcg gtcgttcggc tgcggcgagc ggtatcagct cactcaaagg cggtaatacg 3120
   gttatccaca gaatcagggg ataacgcagg aaagaacatg tgagcaaaag gccagcaaaa 3180
   ggccaggaac cgtaaaaagg ccgcgttgct ggcgtttttc cataggctcc gcccccctga 3240
   cgagcatcac aaaaatcgac gctcaagtca gaggtggcga aacccgacag gactataaag 3300
   ataccaggcg tttccccctg gaagctccct cgtgcgctct cctgttccga ccctgccgct 3360
   taccggatac ctgtccgcct ttctcccttc gggaagcgtg gcgctttctc atagctcacg 3420
   ctgtaggtat ctcagttcgg tgtaggtcgt tcgctccaag ctgggctgtg tgcacgaacc 3480
   ccccgttcag cccgaccgct gcgccttatc cggtaactat cgtcttgagt ccaacccggt 3540
   aagacacgac ttatcgccac tggcagcagc cactggtaac aggattagca gagcgaggta 3600
   tgtaggcggt gctacagagt tcttgaagtg gtggcctaac tacggctaca ctagaaggac 3660
   agtatttggt atctgcgctc tgctgaagcc agttaccttc ggaaaaagag ttggtagctc 3720
   ttgatccggc aaacaaacca ccgctggtag cggtggtttt tttgtttgca agcagcagat 3780
   tacgcgcaga aaaaaaggat ctcaagaaga tcctttgatc ttttctacgg ggtctgacgc 3840
   tcagtggaac gaaaactcac gttaagggat tttggtcatg agattatcaa aaaggatctt 3900
   cacctagatc cttttaaagg ccggccgcgg ccgccatcgg catttctttt tgcgttttta 3960
   tttgttaact gttaattgtc cttgttcaag gatgctgtct ttgacaacag atgttttctt 4020
   gcctttgatg ttcagcagga agctcggcgc aaacgttgat tgtttgtctg cgtagaatcc 4080
   tctgtttgtc atatagcttg taatcacgac attgtttcct ttcgcttgag gtacagcgaa 4140
   gtgtgagtaa gtaaaggtta catcgttagg atcaagatcc atttttaaca caaggccagt 4200
   tttgttcagc ggcttgtatg ggccagttaa agaattagaa acataaccaa gcatgtaaat 4260
   atcgttagac gtaatgccgt caatcgtcat ttttgatccg cgggagtcag tgaacaggta 4320
   ccatttgccg ttcattttaa agacgttcgc gcgttcaatt tcatctgtta ctgtgttaga 4380
   tgcaatcagc ggtttcatca cttttttcag tgtgtaatca tcgtttagct caatcatacc 4440
   gagagcgccg tttgctaact cagccgtgcg ttttttatcg ctttgcagaa gtttttgact 4500
   ttcttgacgg aagaatgatg tgcttttgcc atagtatgct ttgttaaata aagattcttc 4560
   gccttggtag ccatcttcag ttccagtgtt tgcttcaaat actaagtatt tgtggccttt 4620
   atcttctacg tagtgaggat ctctcagcgt atggttgtcg cctgagctgt agttgccttc 4680
   atcgatgaac tgctgtacat tttgatacgt ttttccgtca ccgtcaaaga ttgatttata 4740
   atcctctaca ccgttgatgt tcaaagagct gtctgatgct gatacgttaa cttgtgcagt 4800
   tgtcagtgtt tgtttgccgt aatgtttacc ggagaaatca gtgtagaata aacggatttt 4860
   tccgtcagat gtaaatgtgg ctgaacctga ccattcttgt gtttggtctt ttaggataga 4920
   atcatttgca tcgaatttgt cgctgtcttt aaagacgcgg ccagcgtttt tccagctgtc 4980
   aatagaagtt tcgccgactt tttgatagaa catgtaaatc gatgtgtcat ccgcattttt 5040
   aggatctccg gctaatgcaa agacgatgtg gtagccgtga tagtttgcga cagtgccgtc 5100
   agcgttttgt aatggccagc tgtcccaaac gtccaggcct tttgcagaag agatattttt 5160
   aattgtggac gaatcaaatt cagaaacttg atatttttca tttttttgct gttcagggat 5220
   ttgcagcata tcatggcgtg taatatggga aatgccgtat gtttccttat atggcttttg 5280
   gttcgtttct ttcgcaaacg cttgagttgc gcctcctgcc agcagtgcgg tagtaaaggt 5340
   taatactgtt gcttgttttg caaacttttt gatgttcatc gttcatgtct ccttttttat 5400
   gtactgtgtt agcggtctgc ttcttccagc cctcctgttt gaagatggca agttagttac 5460
   gcacaataaa aaaagaccta aaatatgtaa ggggtgacgc caaagtatac actttgccct 5520
   ttacacattt taggtcttgc ctgctttatc agtaacaaac ccgcgcgatt tacttttcga 5580
   cctcattcta ttagactctc gtttggattg caactggtct attttcctct tttgtttgat 5640
   agaaaatcat aaaaggattt gcagactacg ggcctaaaga actaaaaaat ctatctgttt 5700
   cttttcattc tctgtatttt ttatagtttc tgttgcatgg gcataaagtt gcctttttaa 5760
   tcacaattca gaaaatatca taatatctca tttcactaaa taatagtgaa cggcaggtat 5820
   atgtgatggg ttaaaaagga tcggcggccg ctcgatttaa atc                          5863
<210> 21
   <211> 1224
   <212> DNA
   <213> Künstliche Sequenz
<220>
   <221> CDS
   <222> (1)..(1224)
   <223>
<400> 21
   gtg gct cag cca acc gcc gtc cgt ttg ttc acc agt gaa tct gta act        48
   Met Ala Gln Pro Thr Ala Val Arg Leu Phe Thr Ser Glu Ser Val Thr
   1               5                   10                  15
   gag gga cat cca gac aaa ata tgt gat gct att tcc gat acc att ttg        96
   Glu Gly His Pro Asp Lys Ile Cys Asp Ala Ile Ser Asp Thr Ile Leu
               20                  25                  30
   gac gcg ctg ctc gaa aaa gat ccg cag tcg cgc gtc gca gtg gaa act       144
   Asp Ala Leu Leu Glu Lys Asp Pro Gln Ser Arg Val Ala Val Glu Thr
           35                  40                  45
   gtg gtc acc acc gga atc gtc cat gtt gtt ggc gag gtc cgt acc agc       192
   Val Val Thr Thr Gly Ile Val His Val Val Gly Glu Val Arg Thr Ser
       50                  55                  60
   gct tac gta gag atc cct caa tta gtc cgc aac aag ctc atc gaa atc       240
   Ala Tyr Val Glu Ile Pro Gln Leu Val Arg Asn Lys Leu Ile Glu Ile
   65                  70                  75                  80
   gga ttc aac tcc tct gag gtt gga ttc gac gga cgc acc gct ggc gtc       288
   Gly Phe Asn Ser Ser Glu Val Gly Phe Asp Gly Arg Thr Ala Gly Val
                   85                  90                  95
   tca gta tcc atc ggt gag cag tcc cag gaa atc gct gac ggc gtg gat       336
   Ser Val Ser Ile Gly Glu Gln Ser Gln Glu Ile Ala Asp Gly Val Asp
               100                 105                 110
   aac tcc gac gaa gcc cgc acc aac ggc gac gtt gaa gaa gac gac cgc       384
   Asn Ser Asp Glu Ala Arg Thr Asn Gly Asp Val Glu Glu Asp Asp Arg
           115                 120                 125
   gca ggt gct ggc gac cag ggc ctg atg ttc ggc tac gcc acc aac gaa       432
   Ala Gly Ala Gly Asp Gln Gly Leu Met Phe Gly Tyr Ala Thr Asn Glu
           130                 135                 140
   acc gaa gag tac atg cct ctt cct atc gcg ttg gcg cac cga ctg tca      480
   Thr Glu Glu Tyr Met Pro Leu Pro Ile Ala Leu Ala His Arg Leu Ser
   145                 150                 155                 160
   cgt cgt ctg acc cag gtt cgt aaa gag ggc atc gtt cct cac ctg cgt      528
   Arg Arg Leu Thr Gln Val Arg Lys Glu Gly Ile Val Pro His Leu Arg
                   165                 170                 175
   cca gac gga aaa acc cag gtc acc ttc gca tac gat gcg caa gac cgc      576
   Pro Asp Gly Lys Thr Gln Val Thr Phe Ala Tyr Asp Ala Gln Asp Arg
               180                 185                 190
   cct agc cac ctg gat acc gtt gtc atc tcc acc cag cac gac cca gaa      624
   Pro Ser His Leu Asp Thr Val Val Ile Ser Thr Gln His Asp Pro Glu
           195                 200                 205
   gtt gac cgt gca tgg ttg gaa acc caa ctg cgc gaa cac gtc att gat      672
   Val Asp Arg Ala Trp Leu Glu Thr Gln Leu Arg Glu His Val Ile Asp
       210                 215                 220
   tgg gta atc aaa gac gca ggc att gag gat ctg gca acc ggt gag atc      720
   Trp Val Ile Lys Asp Ala Gly Ile Glu Asp Leu Ala Thr Gly Glu Ile
   225                 230                 235                 240
   acc gtg ttg atc aac cct tca ggt tcc ttc att ctg ggt ggc ccc atg      768
   Thr Val Leu Ile Asn Pro Ser Gly Ser Phe Ile Leu Gly Gly Pro Met
                   245                 250                 255
   ggt gat gcg ggt ctg acc ggc cgc aag atc atc gtg gat acc tac ggt      816
   Gly Asp Ala Gly Leu Thr Gly Arg Lys Ile Ile Val Asp Thr Tyr Gly
               260                 265                 270
   ggc atg gct cgc cat ggt ggt gga gca ttc tcc ggt aag gat cca agc      864
   Gly Met Ala Arg His Gly Gly Gly Ala Phe Ser Gly Lys Asp Pro Ser
           275                 280                 285
   aag gtg gac cgc tct gct gca tac gcc atg cgt tgg gta gca aag aac      912
   Lys Val Asp Arg Ser Ala Ala Tyr Ala Met Arg Trp Val Ala Lys Asn
       290                 295                 300
   atc gtg gca gca ggc ctt gct gat cgc gct gaa gtt cag gtt gca tac      960
   Ile Val Ala Ala Gly Leu Ala Asp Arg Ala Glu Val Gln Val Ala Tyr
   305                 310                 315                 320
   gcc att gga cgc gca aag cca gtc gga ctt tac gtt gaa acc ttt gac     1008
   Ala Ile Gly Arg Ala Lys Pro Val Gly Leu Tyr Val Glu Thr Phe Asp
                   325                 330                 335
   acc aac aag gaa ggc ctg agc gac gag cag att cag gct gcc gtg ttg     1056
   Thr Asn Lys Glu Gly Leu Ser Asp Glu Gln Ile Gln Ala Ala Val Leu
               340                 345                 350
   gag gtc ttt gac ctg cgt cca gca gca att atc cgt gag ctt gat ctg     1104
   Glu Val Phe Asp Leu Arg Pro Ala Ala Ile Ile Arg Glu Leu Asp Leu
           355                 360                 365
   ctt cgt ccg atc tac gct gac act gct gcc tac ggc cac ttt ggt cgc     1152
   Leu Arg Pro Ile Tyr Ala Asp Thr Ala Ala Tyr Gly His Phe Gly Arg
   370 375 380
   act gat ttg gac ctt cct tgg gag gct atc gac cgc gtt gat gaa ctt 1200
   Thr Asp Leu Asp Leu Pro Trp Glu Ala Ile Asp Arg Val Asp Glu Leu
   385 390 395 400
   cgc gca gcc ctc aag ttg gcc taa 1224
   Arg Ala Ala Leu Lys Leu Ala
   405
<210> 22
   <211> 407
   <212> PRT
   <213> Künstliche Sequenz
<400> 22
   Met Ala Gln Pro Thr Ala Val Arg Leu Phe Thr Ser Glu Ser Val Thr
   1 5 10 15
   Glu Gly His Pro Asp Lys Ile Cys Asp Ala Ile Ser Asp Thr Ile Leu
   20 25 30
   Asp Ala Leu Leu Glu Lys Asp Pro Gln Ser Arg Val Ala Val Glu Thr
   35 40 45
   Val Val Thr Thr Gly Ile Val His Val Val Gly Glu Val Arg Thr Ser
   50 55 60
   Ala Tyr Val Glu Ile Pro Gln Leu Val Arg Asn Lys Leu Ile Glu Ile
   65 70 75 80
   Gly Phe Asn Ser Ser Glu Val Gly Phe Asp Gly Arg Thr Ala Gly Val
   85 90 95
   Ser Val Ser Ile Gly Glu Gln Ser Gln Glu Ile Ala Asp Gly Val Asp
   100 105 110
   Asn Ser Asp Glu Ala Arg Thr Asn Gly Asp Val Glu Glu Asp Asp Arg
   115 120 125
   Ala Gly Ala Gly Asp Gln Gly Leu Met Phe Gly Tyr Ala Thr Asn Glu
   130 135 140
   Thr Glu Glu Tyr Met Pro Leu Pro Ile Ala Leu Ala His Arg Leu Ser
   145 150 155 160
   Arg Arg Leu Thr Gln Val Arg Lys Glu Gly Ile Val Pro His Leu Arg
   165 170 175
   Pro Asp Gly Lys Thr Gln Val Thr Phe Ala Tyr Asp Ala Gln Asp Arg
   180 185 190
   Pro Ser His Leu Asp Thr Val Val Ile Ser Thr Gln His Asp Pro Glu
   195 200 205
   Val Asp Arg Ala Trp Leu Glu Thr Gln Leu Arg Glu His Val Ile Asp
   210 215 220
   Trp Val Ile Lys Asp Ala Gly Ile Glu Asp Leu Ala Thr Gly Glu Ile
   225 230 235 240
   Thr Val Leu Ile Asn Pro Ser Gly Ser Phe Ile Leu Gly Gly Pro Met
   245 250 255
   Gly Asp Ala Gly Leu Thr Gly Arg Lys Ile Ile Val Asp Thr Tyr Gly
   260 265 270
   Gly Met Ala Arg His Gly Gly Gly Ala Phe Ser Gly Lys Asp Pro Ser
   275 280 285
   Lys Val Asp Arg Ser Ala Ala Tyr Ala Met Arg Trp Val Ala Lys Asn
   290 295 300
   Ile Val Ala Ala Gly Leu Ala Asp Arg Ala Glu Val Gln Val Ala Tyr
   305 310 315 320
   Ala Ile Gly Arg Ala Lys Pro Val Gly Leu Tyr Val Glu Thr Phe Asp
   325 330 335
   Thr Asn Lys Glu Gly Leu Ser Asp Glu Gln Ile Gln Ala Ala Val Leu
   340 345 350
   Glu Val Phe Asp Leu Arg Pro Ala Ala Ile Ile Arg Glu Leu Asp Leu
   355 360 365
   Leu Arg Pro Ile Tyr Ala Asp Thr Ala Ala Tyr Gly His Phe Gly Arg
   370 375 380
   Thr Asp Leu Asp Leu Pro Trp Glu Ala Ile Asp Arg Val Asp Glu Leu
   385 390 395 400
   Arg Ala Ala Leu Lys Leu Ala
   405
<210> 23
   <211> 9
   <212> PRT
   <213> Künstliche Sequenz
<220>
   <221> MISC_FEATURE
   <222> (2).. (2)
   <223> Xaa=Phe oderTyr
<220>
   <221> MISC_FEATURE
   <222> (3)..(3)
   <223> Xaa=Asp oder Ser
<220>
   <221> MISC_FEATURE
   <222> (4) .. (4)
   <223> Xaa=beliebige Aminosäure
<220>
   <221> MISC_FEATURE
   <222> (5).. (5)
   <223> Xaa=beliebige Aminosäure
<220>
   <221> MISC_FEATURE
   <222> (7) .. (7)
   <223> Xaa=von Cys verschiedene Aminosäure
<220>
   <221> MISC_FEATURE
   <222> (6)..(6)
   <223> Xaa=Ser oder Thr
<220>
   <221> MISC_FEATURE
   <222> (8)..(8)
   <223> Xaa=Gly oder Ala
<400> 23
   Gly Xaa Xaa Xaa Xaa Xaa Xaa Xaa Val
   1 5